# EUROPEAN PATENT APPLICATION

(11) **EP 1 865 071 A1**
(43) Date of publication of application: **12.12.2007**
(21) Application number: 06115230.2
(22) Date of filing: 09.06.2006
(51) Int. Cl.: C12Q 1/68

(54) **Method for early diagnosis of proliferative diabetic retinopathy**

(71) Applicant: Rheinische Friedrich-Wilhelms-Universität Bonn, 53113 Bonn (DE)
(72) Inventor: Golubnitschaja, Olga, Prof. Dr., 53125 Bonn (DE); Holz, Frank, Prof. Dr., 53127 Bonn (DE)
(74) Representative: Helbing, Jörg

(57) **Abstract**

The present invention provides an *ex vivo* method for diagnosis, especially early diagnosis, of proliferative diabetic retinopathy. Furthermore, it provides a kit for performing said method.

## Description

The present invention provides an *ex vivo* method for diagnosis, especially early diagnosis, of proliferative diabetic retinopathy. Furthermore, it provides a kit for performing said method.

### Background of the Invention

Diabetic retinopathy (DRP) is worldwide the leading cause of visual loss in diabetic patients. It can be one of the earliest events observed even in a very young diabetic population. DRP is a disease of the small blood vessels of the retina of the eye. When retinopathy first starts, the tiny blood vessels in the retina become swollen, and they leak a little fluid into the center of the retina. The person's sight may be blurred. This condition is called background retinopathy or non-proliferative DRP. About 80 percent of people with background retinopathy never have serious vision problems, and the disease never goes beyond this first stage. However, if retinopathy progresses, the harm to sight can be more serious. Many new, tiny blood vessels grow out and across the eye (neovascularization). The vessels may break and bleed into the clear gel that fills the center of the eye, blocking vision. Scar tissue may also form near the retina, pulling it away from the back of the eye. This stage is called proliferative retinopathy, and it can lead to impaired vision and even blindness.

A micro vascular disorder is implicated in the pathomechanisms of DRP (Katsumori, K. et al., Diabetes Res. Clin. Pract. 29(3): 195-201 (1995); Akasaka, T. et al., J. Am. Coll. Cardiol. 30(4):935-41 (1997); Oku, H. et al., Invest. Ophthalmol. Vis. Sci. 42(8): 1915-20 (2001)). Abnormalities in the microcirculation observed in diabetes mellitus (DM) patients correlate significantly with the severity of hyperglycemia. However, both the occurrence and the severity of diabetic micro vascular disease vary among individual patients and do not necessarily correlate with the duration of DM (Cheung, A.T. et al., Endocrin. Pract. 7(5):358-363 (2001)). Moreover, even in cases of well-balanced glycemia (e.g. no periodic high-low glucose fluctuations in blood), there are three main subgroups among diabetic patients, namely groups with (1) no visible secondary complications, (2) mild complications and (3) severe complications. Therefore, a predisposition to the development of secondary complications which is independent from hyperglycemia and DM duration has been proposed for DM patients (Bodansky, H.J. et al., Diabetes 31(1):70-4 (1982); Strippoli, G.F. et al., J. Nephrol. 16(4):487-99 (2003)).

The deposition of advanced glycation end products is enhanced in DM and has been linked to the micro vascular disease underlying DRP (Wautier, J.L., and Guillausseau, P.J., Vasc. Med. 3(2):131-7 (1998)). Glycated proteins have receptors on mononuclear blood cells (MBCs) (one kind of leukocytes) and generate reactive oxygen species altering gene expression and modifying cellular targets, such as endothelial cells (Schmidt, A.M. et al., Arterioscler. Thromb. 14(10): 1521-8 (1994)). Modified endothelial cells, further, play the key role in the pathology of both diabetic cardiovasculopathy and nephropathy (Petty, R.G. et al., Diabetes Res. 17(3):115-23 (1991)). This cause-and-effect chain is the reason why the development of proliferative retinopathy is usually the most early and best recognizable pathology among diverse diabetic complications, whilst it is only extremely rarely observed in non-diabetic population. Furthermore, once occurred, the proliferative diabetic retinopathy is usually followed by later diabetic complications such as cardiomyopathy, nephropathy and others. Therefore, the appearance of proliferative retinopathy can be considered as an early indicator for a predisposition for development of secondary complications in diabetic patients. Thus, one of the objectives of present invention is to present a connection between this predisposition and the molecular interplay leading to the occurrence of proliferative retinopathy and further diabetic complications.

Diabetic retinopathy has an underlying inflammatory component, manifesting itself in leukocyte recruitment and up-regulation of genes responsive to inflammatory processes (van Hecke, M.V. et al., Diabetologia 48(7):1300-6 (2005)). Adhesion of leukocytes to the retinal vasculature is one of the earliest events in experimental DM that results in breakdown of the blood-retinal barrier, endothelial cell damage, and capillary non-perfusion (Ogura, Y., Jpn. J. Ophthalmol. 44(3):322-3 (2000); Boeri, D. et al., Diabetes 50(6):1432-9 (2001); Hofman, P. et al., Arch. Ophthalmol. 119(6):861-6 (2001)). By blocking adhesion molecules on the vascular endothelium, the leukocyte-endothelial interactions and concomitantly a retinal atrophy were effectively inhibited in animal models (Ogura, Y., Jpn. J. Ophthalmol. 44(3):322-3 (2000)). This indicates a functional relationship between leukocytes recruitment and development of retinal atrophy.

DRP is currently detected mainly by microscopic, particularly by angiographic methods. Said methods provide no possibility to predict any development of DRP or of any other secondary complications in diabetes. Instead, they just allow assertion of the *status quo.*

Thus, there is a need for a method, which allows a prediction of the risk to develop proliferative DRP and a prediction on the probability of DRP progression. Such risk evaluation is essential for improvement of life quality of the patients, of the blindness emergency response and of prediction and prevention of further secondary diabetic complications besides DRP.

### Summary of the invention

Present invention is based on the central role of activated leukocytes and of matrix metalloproteinases in the pathophysiology of DRP. It presents a functional link between specific alterations in gene expression of leukocytes, the increased activity of tissue-remodeling matrix metalloproteinases in serum, and the proliferative retinopathy in DM.

Differential gene expression in leukocytes was analyzed in order to identify molecular markers specifically expressed during progression and possibly even during development of the disease. The identified genes, which are involved in retinal angiogenesis also represent potential targets for medicaments, which suppress retinal neovascularization in DM.

Furthermore, the activity of MMP-9 and MMP-2 was analyzed, since an activation of these genes is known to play a key role in tissue remodeling and neovascularization and is supposed to be involved in vascular pathomechanisms conditioned by hyperglycemia (Uemura, S. et al., Circ. Res. 88(12):1291-8 (2001); Mene, P. et al., Nephrol. Dial. Transplant. 16(5):913-22 (2001)).

Thus, the present invention provides
(1) a method for evaluation of the risk of a diabetes mellitus (DM) patient to develop diabetic retinopathy (DRP), for early diagnosis of diabetic retinopathy, for determination of the progression risk of an existing diabetic retinopathy, and for determination of the risk to develop secondary complications to DM, which comprises
   (a) determining *ex vivo* in a body fluid sample from the patient at least one, preferably at least three, more preferably at least five parameter(s) selected from the group consisting of (i) the expression level of one or more proteins of the group consisting of NF-kappaB, XIAP, TIMP-1, TIMP-2 and Ly-GDI, (ii) the activity of the matrix metalloproteinases MMP-2 and/or MMP-9, and (iii) the ratio MMP-2 activity:TIMP-2 expression level (MMP-2:TIMP-2) and/or MMP-9 activity:TIMP-1 expression level (MMP-9:TIMP-1);
   (b) comparing the parameter(s) determined in step (a) with the corresponding mean value(s) of a non-diabetic control group or with the corresponding earlier value(s) of the same patient ("reverence value") and identifying of increased or decreased parameter level(s); and
   (c) identifying one or more risk factors based on said increased or decreased parameter level(s);
(2) a preferred embodiment of (1) above, wherein in step (c) said risk factors are selected from the group consisting of (A) increased Ly-GDI expression, increased MMP-2 activity, increased ratio MMP-2:TIMP-2, increased MMP-9 activity, increased ratio MMP-9:TIMP-1, and (B) decreased TIMP-1 expression, decreased NF-kappaB expression, decreased XIAP expression, and at most 2 times increased, preferably decreased TIMP-2 expression, in comparison to the reference value; and
(3) a kit for performing the method of (1) or (2) above comprising reagents and/or devices for
   (i) detecting the expression of the genes listed above, and/or
   (ii) determining the activity of MMP-2 and/or MMP-9.
A patient showing at least one, preferably more than one of the risk factors listed under (2) has an increased risk to develop DRP or that an existing DRP will progress to later stages, especially to later proliferative stages, or that secondary complications will develop.

### Short Description of the Figures

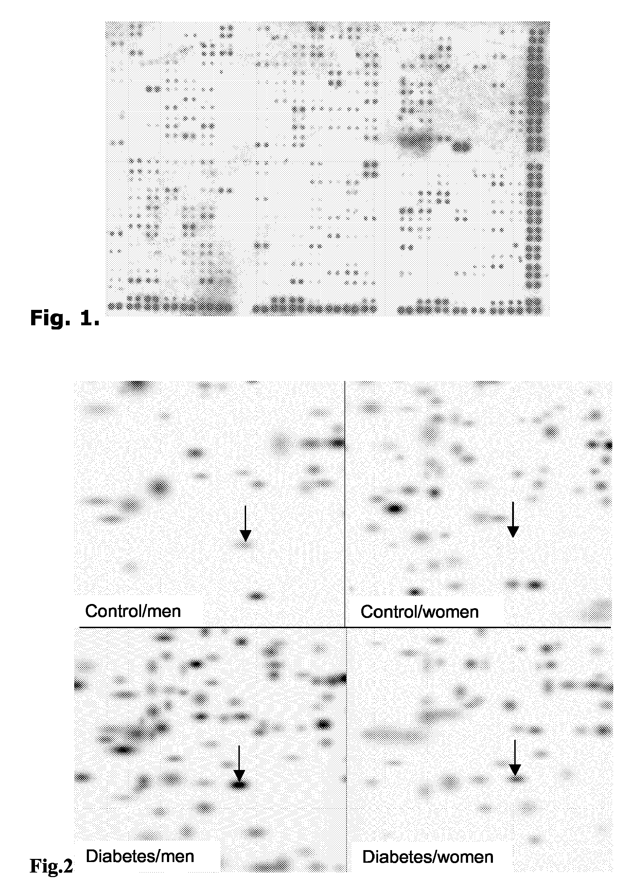

Fig. 1: Expression Array ("Atlas Array") image of cDNA from pooled mRNA of 13 diabetic patients (subject information see example 1, Pilot Study; cDNA synthesis see example 3). The use of cDNA from pooled mRNA allows the detection of general differences between patient and control groups and equalizes individual differences between the subjects. The DNA array comprised double-spotted probes for altogether 588 different cardiovascular diseases specific genes. See Example 4. The differentially expressed genes are listed in Tab. 1.

Fig. 2: 2D-PAGE of total protein from circulating leukocytes of diabetic female and male patients and controls (see example 5A). Arrow: position of Ly-GDI protein spot. Ly-GDI expression is enhanced in diabetic men and women (lower part) in comparison to samples of non-diabetic patients.

**Sequence Listing - Free Text**

| | |
|---|---|
| SEQ ID NO: | Description |
| 1 | DNA encoding human beta actin |
| 2 | human beta actin |
| 3 | DNA encoding human XIAP |
| 4 | human XIAP |
| 5 | DNA encoding human NF-kappaB |
| 6 | human NF-kappaB |
| 7 | DNA encoding human TIMP-2 |
| 8 | human TIMP-2 |
| 9 | DNA encoding human TIMP-1 |
| 10 | human TIMP-1 |
| 11 | DNA encoding human MMP-2 |
| 12 | human MMP-2 |
| 13 | DNA encoding human MMP-9 |
| 14 | human MMP-9 |
| 15 | DNA encoding human Ly-GDI |
| 16 | human Ly-GDI |
| 17 to 26 | primers (compare example 6) |

### Definitions

In the context of present invention, the following abbreviations, terms and definitions are used:
Abbreviations: DRP, diabetic retinopathy; DM, Diabetes mellitus; XIAP, X-linked inhibitor of apoptosis protein, or expression level of said protein; MMP-9, matrix metalloproteinase 9, or the activity level thereof; MMP-2, matrix metalloproteinase 2, or activity level thereof; TIMP-1, tissue inhibitor of metalloproteinase 1, or expression level of said inhibitor; TIMP-2 tissue inhibitor of metalloproteinase 2, or expression level of said inhibitor; NF-kappaB, NF-kappaB or expression level of NF-kappaB; Ly-GDI, lymphoid-specific guanosine diphosphate (GDP) dissociation inhibitor, or expression level of said inhibitor.
The term "reference value" pertains to the mean value of a respective parameter determined in a control group of non-diabetic subjects or to the corresponding value of the same patient, which was determined by the same detection method before DRP set in. The control group of non-diabetic subjects preferably consists of at least 5 subjects, more preferably 10 subjects. Alternatively, reference values for non-diabetic subjects can be taken from literature.
"Relative units" (r.u.) is a *terminus technicus* used for gene expression levels (both transcription and translation) as well as zymography results in molecular biology.
The term "enhanced" pertains to expression or activity levels which are higher than that in the control group (i.e. the reference value), preferably at least two times the mean value of the control group ("slightly increased"). It also encompasses "high" expression and activity levels, namely levels, which are increased several times ("highly increased"), i.e. more than two times, preferably more than three times compared to the mean values of the control group.
"DRP" is diabetic retinopathy. "Proliferative" DRP in the context of present invention means a DRP which has passed the background DRP stadium and is characterized by a diffuse image instead of clearly formed micro-vessels at angiographic detection. A "highly proliferative DRP" is additionally characterized by a diffuse image not only in the area of micro-vessels, but even in the area of macro-vessels.
   The system of "DRP classes" used in present invention is based on the following classification: class I (proliferation "-", "(+)"), class II (proliferation "+", "+(+)"), class III (proliferation "++", "+++", "++++"). Therein, "+" and "-" correlate to the following symptoms:
   "-", "(+)": angiography images similar to those of non-diabetic controls without retinopathy;
   "+ ", "+(+)": no microcirculation detectable in angiography images;
   "++": diffuse image typical for neovascularization of micro-vessels with increasing severity grade;
   "+++", "++++": diffuse image typical for neovascularization of macro-vessels with increasing severity grade.
   Thus, "proliferative DRP" corresponds to class II, "highly proliferative DRP" corresponds to class III, and "no active proliferative DRP" corresponds to class I.
"Risk" means a patient's chance to develop a disease, especially proliferative DRP, or secondary complications, especially secondary complications typical for DM.
A "risk factor" in the context of present application is a condition, which increases the probability that a patient develops a disease, especially proliferative DRP. Risk factors do not cause the disease, and not everyone who has a risk factor will get the disease. However, risk factors help a medical practitioner to focus risk reduction efforts on the people who are most "at risk" for a disease, especially for proliferative DRP. Particularly, the summarized risk factors presented in Tab. 3 provide the most valuable information for a calculation of the probability that a patient will develop proliferative DRP. Thus, a useful risk factor to prognose development of proliferative DRP is characterized by a correlation of its alteration in comparison to a healthy control group ("disease specific alteration") with disease severity. The same applies *mutatis mutandis* to "risk factors" for DRP progression and for development of further secondary complications.
A "secondary disease" is a disease that follows and results from an earlier disease. In the context of present invention, said earlier disease is DM. Said secondary diseases are especially DRP and other cardiovascular complications observed in DM patients.
A "secondary complication" is a secondary disease, an accident, or a negative reaction occurring during the course of an illness and usually aggravating the illness. Particular secondary complications connected to DRP and/or DM are disturbed microcirculation, pathologic remodelling of the vascular system, and possibly even silent myocardial infarcts.
"Expression level" of a protein means its level of expression. It may be determined on the mRNA or protein level. Preferably, the expression level is determined by quantification of mRNA, including quantification by detection with fluorescent reporter molecules and Real-time-PCR, especially PCR using intercalation dye/fluorescent reporter molecule detection or molecular beacons based PCR.

### Detailed Description of the Invention

The present invention provides an *ex vivo* method for diagnosis and prognosis of proliferative DRP. Said method is based on gene expression of a set of molecular markers and on the activity of matrix metalloproteinases.

In one preferred aspect of embodiment (2) of the present invention, the patient shows more than one, preferably at least four, more preferably at least six of the risk factors. Such patient has an increased risk to develop DRP or that an existing DRP will progress to later stages, especially to later proliferative stages, or that secondary complications will develop.

In the method of embodiment (1) or (2), preferably a risk factor which is shown by the patient is an increased ratio MMP-9:TIMP-1. This is especially important if only a few of the parameters listed under embodiment (1) can be measured. MMP-9:TIMP-1 is the most reliant indicator for an increased risk of a DM patient to develop DRP or to progress in DRP.

Furthermore preferred is the use of whole blood or a blood component as the body fluid in the method of embodiments (1) and (2). More preferably, a blood component is used which is either circulating leukocytes, blood plasma or blood serum, or a combination thereof. Most preferred is the use of blood serum or plasma for determination of MMP activity, and of circulating leukocytes for determination of the expression levels listed under (i) in embodiment (1) above.

The determination steps of the method according to the present invention are preferably performed by DNA or RNA detection, more preferably by mRNA detection, for determination of gene expression levels and by zymography or ELISA for determination of MMP activity.

One preferred aspect of the present invention is a method for evaluation of the risk of DRP progression in all DRP stages. A second preferred aspect of the present invention is a method for evaluation of the risk of DRP development in diabetic patients with no retinopathy detected. Both of said methods comprise the determination of one or more, preferably of all, of the parameters of the group consisting of MMP-9 activity, NF-kappaB expression level, TIMP-1 expression level, Ly-GDI expression level, XIAP expression level and the calculation of the ratio of MMP-9:TIMP-1. In a preferred aspect of said methods, one or more of
(i) a ratio MMP-9:TIMP-1 which is at least 1.5 times higher than the reference value;
(ii) a MMP-9 activity level which is at least 1.3 times higher than the reference value;
(iii) a NF-kappaB expression level which is at least 1.5 times lower than the reference value;
(iv) a Ly-GDI expression level which is higher than the reference value; and
(v) a XIAP expression level which is at least 1.5 times lower than the reference value
indicate a risk of DRP development or progression from nonproliferative to proliferative DRP.

In an even more preferred aspect, said methods comprise the determination of one or more, preferably of all, of the parameters of the group consisting of MMP-9 activity, NF-kappaB expression level, TIMP-1 expression level and the calculation of the ratio of MMP-9:TIMP-1. In cases where the XIAP and/or Ly-GDI expression level is determined in addition to one or more of said latter parameters,
(i) a Ly-GDI expression level which is higher than the reference value; and
(ii) a XIAP expression level which is at least 1.5 times lower than the reference value
is an additional indicator for the risk of DRP progression, especially for the risk of DRP progression from nonproliferative to proliferative DRP.

A third preferred aspect of the present invention is a method for evaluation of the risk of DRP progression specifically in late proliferative DRP stages, especially from proliferative (class II) to highly proliferative (class III) DRP. Said method comprises the determination of one or more, preferably of all, of the parameters of the group consisting of MMP-9 and MMP-2 activity, TIMP-1 expression level, TIMP-2 expression level, NF-kappaB expression level and the calculation of the ratio MMP-2:TIMP-2 and MMP-9:TIMP-1. In a preferred aspect of said method, one or more of
(i) a TIMP-2 level which is equal or less than two times the reference value, preferably is lower than the reference value;
(ii) a TIMP-1 level which is at least 0.5 times lower than the reference value;
(iii) a MMP-2 activity level which is at least 1.5 times higher than the reference value;
(iv) a ratio MMP-2:TIMP-2 which is at least 1.5 times higher than the reference value;
(v) a MMP-9 activity level which is at least 1.5 times higher than the reference value;
(vi) a ratio MMP-9:TIMP-1 which is at least 2.5 times higher than the reference value; and
(vii) a NFkappaB expression level which is at least 3 times lower than the reference value
indicate a risk of DRP progression from proliferative DRP to highly proliferative DRP.

In an even more preferred aspect, said method comprises the determination of one or more, preferably of all, of the parameters of the group consisting of MMP-2 activity, TIMP-1 expression level, TIMP-2 expression level and the calculation of the ratio MMP-2:TIMP-2 and MMP-9:TIMP-1. In cases where the NF-kappaB expression level and/or MMP-9 activity level is determined in addition to one or more of said latter parameters,
(i) a NFkappaB expression level which is at least 3 times lower than the reference value; and/or
(ii) a MMP-9 activity level which is at least 1.5 times higher than the reference value
is an additional indicator for the risk of DRP progression from proliferative DRP to highly proliferative DRP.

The kit of embodiment (3) preferably contains reagents and/or devices for
(i) detecting the expression of the proteins listed in embodiment (1) above by "Real-Time"-PCR, especially by primer or molecular beacons based PCR, and/or
(ii) determining the activity of MMP-2 and/or MMP-9 by means to measure the gelatinase activity of MMP-2 and MMP-9, by zymography, or by ELISA.

A comparative investigation of gene expression in circulating leukocytes isolated from DM-patients with *versus* without proliferative retinopathy as well as *versus* non-diabetic controls was performed (Examples 2 to 6). In addition, the activity of two matrix metalloproteinases (MMP-2 and MMP-9) in the serum of patients was measured (Example 7), since an activation of these enzymes is known to play a key role in tissue remodeling and neovascularization (Uemura, S. et al., Circ. Res. 22;88(12):1291-8 (2001); Mene, P. et al., Nephrol. Dial. Transplant. 16(5):913-22 (2001)).

The study consisted of three main parts:
A. Pilot study: differential gene expression analysis comparing transcriptional pools isolated from peripheral leukocytes of 13 diabetic patients with those of 11 non-diabetic controls using a DNA array technique (see example 1, Tab. 4 for information on the individuals);
B. verification of the differential expression of genes selected in part A by a quantification of the specific expression rates on the transcriptional level using real-time-PCR and on the level of protein products using both Western blot and zymography; for this aim newly recruited patients (38 subjects) *versus* non-diabetic pools (22 subjects) were tested (Example 1, Tab. 5, Main Study);
C. statistical analysis of the differences in terms of correlation between individual gene expression rates and enzymatic activities, respectively, and the severity grade of proliferative retinopathy (active neovascularization of retina, determined by angiography).

The hybridization of labeled cDNAs to a DNA array specific for human cardiovascular diseases (Human Cardiovascular Array; Atlas) with altogether 588 cardiovascular diseases relevant genes (Fig. 1, Example 4) revealed a differential expression of 152 genes in leukocytes of the DM-group (13 subjects) which were either activated or suppressed *versus* the non-diabetic group (11 subjects) (Tab. 1) in study part A (pilot study).

**Tab. 1:Differentially expressed genes in leukocytes of DM patients in the pilot study; Position: probe position on Atlas Array; Block: Atlas Array blocks**

| **Position** | **Name** | **Group** |
|---|---|---|
| | **BLOCK A** | |
| A02b | ras-related protein RAP-1A; C21KG; | G Proteins |
| | KREV-1 protein; GTP-binding protein SMG-p21A; G-22K | Oncogenes & Tumor Suppressors |
| A02f | TYRO3 tyrosine-protein kinase receptor; RSE; SKY; DTK | Oncogenes & Tumor Suppressors Intracellular Transducers, Effectors & Modulators Cell Receptors (by Ligands) Protein Kinase Receptors |
| A03c | neogenin | Oncogenes & Tumor Suppressors Growth Factor & Chemokine Receptors Other Receptors (by Activities) |
| A03h | cyclin D1 (CCND1); parathyroid adenomatosis protein 1 (PRAD1) | Cyclins |
| A03j | cyclin-dependent kinase 9 (CDK9); cell division protein kinase 9; PITALRE; cell division cycle protein 2-like 4 (CDC2L4) | Cell Cycle-Regulating Kinases Intracellular Kinase Network Members |
| A031 | prothymosin alpha (PTMA); thymosin alpha (TMSA) | Other Cell Cycle Proteins Intracellular Transducers, Effectors & Modulators Chromatin Proteins |
| A03n | G protein-activated inward rectifier potassium channel 2 (GIRK2); KATP-2; BIR1; KIR32 | Ligand-Gated Ion Channels Cell Signaling & Extracellular Communication Proteins |
| A04k | cyclin-dependent kinase inhibitor 2A (CDKN2A); INK4A; CMM2; MLM; multiple tumor suppressor 1 (MTS1) | CDK Inhibitors Kinase Activators & Inhibitors Oncogenes & Tumor Suppressors |
| A05b | transforming growth factor beta 3 (TGF-beta3; TGFB3) | Growth Factors, Cytokines & Chemokines Oncogenes & Tumor Suppressors |
| A05k | cyclin-dependent kinase 4 inhibitor 2D (CDKN2D); p19-INK4D | CDK Inhibitors Kinase Activators & Inhibitors Oncogenes & Tumor Suppressors |
| A06a | p53-binding mouse double minute 2 homolog (MDM2) | Oncogenes & Tumor Suppressors; Intracellular Transducers, Effectors & Modulators; Apoptosis-Associated Proteins; Cell Cycle-Related Proteins |
| A07c | elk1 ets-related proto-oncogene | Oncogenes & Tumor Suppressors Transcription Activators & Repressors Intracellular Transducers, Effectors & Modulators |
| A07d | FOS-related antigen 1 (FRA1); FOS-like antigen 1 (FOSL1) | Oncogenes & Tumor Suppressors Transcription Activators & Repressors Intracellular Transducers, Effectors & Modulators |
| A07f | yes proto-oncogene; YES1 | Oncogenes & Tumor Suppressors Intracellular Adaptors & Receptor-Associated Proteins |
| A07l | ribosomal protein S19 (RPS19) | Other Cell Cycle Proteins Ribosomal Proteins Intracellular Transducers, Effectors & Modulators |
| A07n | calcium-activated potassium channel HSK1 | Ligand-Gated Ion Channels |
| A08d | v-erbA homolog-like 2 (EAR2); nuclear receptor subfamily 2 group F member 6 (NR2F6) | Oncogenes & Tumor Suppressors Transcription Activators & Repressors Intracellular Transducers, Effectors & Modulators |
| A08e | met proto-oncogene; hepatocyte growth factor receptor (HGF receptor; HGFR) | Oncogenes & Tumor Suppressors Intracellular Transducers, Effectors & Modulators Growth Factor & Chemokine Receptors Protein Kinase Receptors |
| A08f | fes proto-oncogene | Oncogenes & Tumor Suppressors Intracellular Adaptors & Receptor-Associated Proteins |
| A08i | mitogen-activated protein kinase 3 (MAP kinase 3; MAPK3; PRKM3); extracellular signal-regulated kinase 1 (ERK1); ERT2 | Cell Cycle-Regulating Kinases Intracellular Kinase Network Members |
| A09i | mitogen-activated protein kinase 1 (MAP kinase 1; MAPK1; PRKM1); extracellular signal-regulated kinase 2 (ERK2) | Cell Cycle-Regulating Kinases Intracellular Kinase Network Members |
| A09m | solute carrier family 7 member 5 (SLC7A5); membrane protein E16 (MPE16) | Facilitated Diffusion Proteins |
| A10e | epidermal growth factor receptor (EGF receptor; EGFR); erbB proto-oncogene | Oncogenes & Tumor Suppressors Intracellular Transducers, Effectors & Modulators Growth Factor & Chemokine Receptors Protein Kinase Receptors |
| A11a | retinoblastoma-associated protein 1 (RB1); phosphoprotein110 (pp110) | Oncogenes & Tumor SuppressorsCell Cycle-Related ProteinsIntracellular Transducers, Effectors & Modulators |
| A11j | CDC-like kinase 3 (CLK3) | Cell Cycle-Regulating Kinases Intracellular Kinase Network Members |
| A11m | aquaporin 9 (AQP9) | Facilitated Diffusion Proteins |
| A13e | erbB4 proto-oncogene; HER4; neuregulin receptor | Oncogenes & Tumor Suppressors Intracellular Transducers, Effectors & Modulators Growth Factor & Chemokine Receptors Protein Kinase Receptors |
| A14a | colorectal mutant cancer protein (MCC) | Oncogenes & Tumor Suppressors |

| | **BLOCK B** | |
|---|---|---|
| B05d | CC chemokine receptor type 1 (CMKBR1; CCCKR1; CCR1); macrophage inflammatory protein 1 alpha receptor (MIP1-alpha receptor; MIP1-alphaR) | Intracellular Transducers, Effectors & Modulators Growth Factor & Chemokine Receptors G Protein-Coupled Receptors |
| B05h | tyrosine kinase 2 (TYK2) | Intracellular Kinase Network Members |
| B05j | mitogen-activated protein kinase 9 (MAP kinase 9; MAPK9; PRKM9); c-jun N-terminal kinase 2 (JNK2) | Intracellular Kinase Network Members Stress Response Proteins |
| B05k | non-receptor tyrosine kinase 1 (TNK1) | Intracellular Kinase Network Members |
| B051 | ribosomal protein S6 kinase II alpha 3 (S6KII-alpha 3); ribosomal S6 kinase 2 (RSK2); insulin-stimulated protein kinase 1 (ISPK1) | Intracellular Kinase Network Members Cell Cycle-Regulating Kinases |
| B06b | sodium/potassium-transporting ATPase isoform 2 beta polypeptide 2 (Na+/K+ ATPase 2 beta 2; ATP2B2) | ATPase Transporters Cell Signaling & Extracellular Communication Proteins |
| B07f | G protein-coupled receptor HM74 | Intracellular Transducers, Effectors & Modulators Other Receptors (by Ligands) Orphan Receptors G Protein-Coupled Receptors |
| B07g | Ink adaptor protein | Intracellular Adaptors & Receptor-Associated Proteins Cell Cycle-Related Proteins |
| B08c | thyroxine-binding globulin; T4-binding globulin | Extracellular Transporters & Carrier Proteins |
| B08h | LIM domain kinase 1 (LIMK1) | Intracellular Kinase Network Members |
| B091 | phosphatidylinositol-specific phospholipase C beta 3 (PLC-beta 3; PLCB3) | Phospholipases & Phosphoinositol Kinases |
| B11a | sodium/glucose cotransporter 2; (Na+/glucose cotransporter 2); low-affinity sodium-glucose cotransporter | Symporters & Antiporters |
| B11b | adrenoleukodystrophy protein (ALDP); X-linked ALD | Cell Signaling & Extracellular Communication Proteins ABC Transporters |
| B11m | guanine nucleotide-binding protein alpha 13 subunit (GNA13) | G Proteins |
| B12a | kidney oligopeptide transporter; kidney H+/peptide cotransporter | Symporters & Antiporters |
| B12b | ATP-binding cassette subfamily G member 1 (ABCG1); ATP-binding cassette 8 (ABC8); white homolog | ABC Transporters |
| B12j | tec tyrosine-protein kinase | Intracellular Kinase Network Members |
| B13d | CXC chemokine receptor type 4 (CXCR4); stromal cell-derived factor 1 receptor (SDF1 receptor); fusin; leukocyte-derived seven transmembrane domain receptor (LESTR); LCR1 | Intracellular Transducers, Effectors & Modulators Growth Factor & Chemokine Receptors G Protein-Coupled Receptors |
| B14d | ephrin type A receptor 1 (EPHA1) | Intracellular Transducers, Effectors & Modulators Growth Factor & Chemokine Receptors Protein Kinase Receptors |
| B14j | Janus tyrosine-protein kinase 2 (JAK2) | Intracellular Kinase Network Members |
| B14k | protein tyrosine kinase 2 beta (PTK2B; PYK2); proline-rich tyrosine kinase 2; protein kinase B (PKB); cell adhesion kinase beta (CAK-beta; CAKB); focal adhesion kinase 2 (FADK2; FAK2) | Intracellular Kinase Network Members |
| B14n | dual-specificity protein phosphatase 9; mitogen-activated protein kinase phosphatase 4 (MAP kinase phosphatase 4 (MKP4) | Cell Cycle-Related Proteins Intracellular Protein Phosphatases |

| | **BLOCK C** | |
|---|---|---|
| C01f | FKBP-rapamycin associated protein (FRAP); rapamycin target protein | Other Intracellular Transducers, Effectors & Modulators Cell Cycle-Related Proteins |
| C01g | retinoic acid receptor beta (RXR-beta; RXRB) | Death ReceptorsTranscription Activators & RepressorsIntracellular Transducers, Effectors & ModulatorsHormone ReceptorsNuclear Receptors |
| C01j | granzyme A (GZMA); cytotoxic T-lymphocyte-associated protein 3 (CTLA3); Hanukkah factor (H factor; HF); fragmentin 1 | Granzymes Serine Proteases |
| C02c | retinal guanylyl cyclase 1 (RETGC-1); retinal guanylate cyclase 2D; rod outer segment membrane guanylate cyclase (ROS-GC) | Adenylate/Guanylate Cyclases & Diesterases |
| C02g | WSL protein + TRAMP + Apo-3 + death domain receptor 3 (DDR3) | Death Receptors Growth Factor & Chemokine Receptors Death Domain Receptors |
| C02k | nitric oxide synthase 2A (NOS2A); inducible hepatocyte NOS (HEP-NOS) | Other Apoptosis-Associated Proteins Cell Signaling & Extracellular Communication Proteins |
| C03d | cAMP-response element binding protein 1 (CREB1) | Transcription Activators & Repressors Intracellular Transducers, Effectors & Modulators |
| C03e | linker for activation of T-cells (LAT) | Kinase Activators & Inhibitors Cell Cycle-Related Proteins |
| C04h | caspase 2 (CASP2); neural precursor cell-expressed developmentally down-regulated protein 2 (NEDD2); ICH1 | Caspases Cysteine Proteases |
| C04k | inhibitor of apoptosis protein 3 (API3; IAP3); X-linked inhibitor of apoptosis protein (X-linked IAP; XIAP); IAP-like protein (HILP) | Other Apoptosis-Associated Proteins |
| C05m | minichromosome maintenance deficient 7 homolog (MCM7); MCM2; CDC47 homolog | DNA Polymerases, Replication Factors & Topoisomerases Other Cell Cycle Proteins |
| C06c | neurogranin (NRGN); RC3 | Calcium-Binding Proteins Cell Signaling & Extracellular Communication Proteins |
| C06e | stratifin (SFN); 14-3-3 protein sigma; epithelial cell marker protein 1; HME1 | Kinase Activators & Inhibitors Oncogenes & Tumor Suppressors |
| C06k | cytochrome P450 reductase | Other Apoptosis-Associated Proteins Stress Response Proteins |
| C07c | recoverin; cancer-associated retinopathy protein (CAR protein) | Calcium-Binding Proteins |
| C08f | tumor necrosis factor alpha (TNF-alpha; TNFA); TNF superfamily member 2 (TNFSF2); cachectin | Death Receptor LigandsGrowth Factors, Cytokines & Chemokines |
| C09g | adenosine A1 receptor (ADORA1) | Death Receptors Cell Signaling & Extracellular Communication Proteins Other Receptors (by Ligands) G Protein-Coupled Receptors |
| C09j | IEX-1L anti-death protein; PRG-1; DIF-2 | Other Apoptosis-Associated Proteins |
| C10l | activator 1 40-kDa subunit (A1 40-kDa subunit); replication factor C 40-kDa subunit (RFC40); RFC2 | DNA Polymerases, Replication Factors & Topoisomerases |
| C11h | caspase 10 (CASP10); MCH4; ICE-like apoptotic protease 4 (ICE-LAP4); FADD-like ICE 2 (FLICE2) | Caspases Cysteine Proteases |
| C12j | growth arrest & DNA damage-inducible protein 153 (GADD153); DNA damage-inducible transcript 3 (DDIT3); C/EBP homologous protein (CHOP) | Other Apoptosis-Associated Proteins DNA Synthesis, Recombination & Repair Proteins Oncogenes & Tumor Suppressors Stress Response Proteins |
| C12m | ATP-dependent DNA ligase I (LIG1); polydeoxyribonucleotide synthase | DNA Damage Repair Proteins & Ligases DNA Polymerases, Replication Factors & Topoisomerases Stress Response Proteins |
| C13b | cAMP-dependent 3',5'-cyclic phosphodiesterase 4D (PDE43); DPDE3 | Adenylate/Guanylate Cyclases & Diesterases |
| C14g | tumor necrosis factor receptor 1-associated death domain protein (TNFR1-associated death domain protein; TRADD) | Death Receptor-Associated Proteins & Adaptors Intracellular Adaptors & Receptor-Associated Proteins |
| C14j | early response protein NAK1; TR3 orphan receptor | Other Apoptosis-Associated Proteins Transcription Activators & Repressors Hormone Receptors Orphan Receptors Nuclear Receptors |

| | **BLOCK D** | |
|---|---|---|
| D01a | X-ray repair-complementing defective repair in Chinese hamster cells 1 (XRCC1) | DNA Damage Repair Proteins & Ligases Stress Response Proteins |
| D01e | nerve growth factor receptor (NGF receptor; NGFR) | Cell Signaling & Extracellular Communication Proteins Neurotransmitter Receptors Death Domain Receptors |
| D02c | somatostatin receptor 2 (SSTR2; SS2R); SRIF1 | Cell Signaling & Extracellular Communication Proteins Neurotransmitter Receptors G Protein-Coupled Receptors |
| D02d | 5-hydroxytryptamine receptor 3A receptor (HTR3A); serotonin receptor | Cell Signaling & Extracellular Communication Proteins Neurotransmitter Receptors Voltage-Gated Ion Channels |
| D02h | brain-specific polypeptide PEP-19; brain-specific antigen PCP-4 | Cell Signaling & Extracellular Communication Proteins |
| | | Cell Surface Antigens |
| D03j | nuclear transcription factor Y gamma (NFYC); CCAAT transcription-binding factor gamma subunit; HSM1 | Basic Transcription Factors |
| D05d | GABA-B receptor 1A subunit (GABA-BR1A) | Cell Signaling & Extracellular Communication Proteins Neurotransmitter Receptors Ligand-Gated Ion Channels Other Receptors (by Activities) |
| D05k | microphthalmia-associated transcription factor (MITF) | Transcription Activators & Repressors |
| D05m | nuclear factor NF-kappa-B p100 subunit; nuclear factor NF-kappa-B p52 subunit; H2TF1; oncogene lyt-10 | Transcription Activators & Repressors |
| D06f | preprotachykinin beta (beta-PPT); substance K; neuromedin L | Cell Signaling & Extracellular Communication Proteins Neuropeptides |
| D06j | TIS11B protein; butyrate response factor 1 (BRF1); EGF response factor 1 (ERF1) | Transcription Activators & Repressors |
| D09a | V(D)J recombination activating protein 2 (RAG2) | Recombination Proteins |
| D09m | basic transcription element-binding protein 2 (BTEB2); GC-box binding protein 2 | Basic Transcription Factors |
| D10d | glutamate receptor subunit epsilon 2 (GRIN2); N-methyl D-aspartate receptor subtype 2B (NMDAR2B; NR2B) | Cell Signaling & Extracellular Communication ProteinsNeurotransmitter ReceptorsLigand-Gated Ion ChannelsOther Receptors (by Activities) |
| D10g | neuromodulin; growth-associated protein 43 (GAP43); protein F1; calmodulin-binding protein p57 | Cell Signaling & Extracellular Communication Proteins Intracellular Transducers, Effectors & Modulators |
| D10i | atrophin-1; dentatorubral-pallidoluysian atrophy protein (DRPLA) | Cell Signaling & Extracellular Communication Proteins Functionally Unclassified Proteins |
| D13i | Kallmann syndrome protein (KAL); adhesion molecule-like X-linked | Cell Signaling & Extracellular Communication Proteins Other Cell Adhesion Proteins |
| D13j | serum response factor (SRF) | Transcription Activators & Repressors |
| D13k | B-cell lymphoma protein 6 (BCL6); BCL5; zinc finger protein 51 (ZNF51); lymphoma-associated zinc finger gene on chromosome 3 (LAZ3) | Transcription Activators & Repressors |

| | **BLOCK E** | |
|---|---|---|
| E01f | integrase interactor 1 (INI1) | Transcription Activators & Repressors |
| E02e | activating transcription factor 4 (ATF4); tax-responsive enhancer element B67 (TAXREB67); cAMP-responsive element-binding protein 2 (CREB2) | Transcription Activators & Repressors |
| E02h | corneodesmosin (CDSN); S protein | Other Cell Adhesion Proteins |
| E02k | complement component 5 receptor 1 | Growth Factor & Chemokine |
| | (C5R1); C5a anaphylatoxin receptor (C5AR); CD88 antigen | Receptors Cell Receptors (by Ligands) |
| E03d | transcription initiation factor IID 31-kDa subunit (TFIID); TATA-box-binding protein-associated factor RNA polymerase II G 32-kDa subunit (TAFII32; TAF2G); TAFII31 | RNA Polymerase |
| E03i | integrin beta 2 (ITGB2); cell surface adhesion glycoproteins LFA-1/CR3/p150,95 beta subunit; CD18 antigen; complement receptor C3 beta subunit | Cell-Cell Adhesion Receptors |
| E03k | neuromedin B receptor (NMBR) | Growth Factor & Chemokine ReceptorsCell Receptors (by Ligands) |
| E05a | transcription factor E2-alpha (E2A); immunoglobulin enhancer binding factor E12; transcription factor-3 (TCF3) | Transcription Activators & Repressors |
| E05e | zinc finger protein 161 (ZNF161); putative transcription activator DB1 | Transcription Activators & Repressors |
| E06c | homeobox protein A1 (HOXA1); HOX1F; homeobox 1 cluster gene 6 homolog (HOX1.6) | CDK Inhibitors |
| E06e | albumin D box-binding protein; D-binding protein (DBP); TAXREB302 | Transcription Activators & Repressors |
| E07b | fli1 proto-oncogene | Transcription Activators & Repressors |
| E07e | zinc finger protein 91 (ZNF92); HPF7; HTF10 | Transcription Activators & Repressors |
| E08b | paired box protein 5 (PAX5); B-cell lineage-specific activator protein (BSAP) | Basic Transcription Factors |
| E08j | erythropoietin receptor (EPOR) | Growth Factor & Chemokine Receptors Cell Receptors (by Ligands) |
| E09b | special AT-rich sequence-binding protein 1 (SATB1) | CDK Inhibitors |
| E09d | zinc-finger DNA-binding protein | Transcription Activators & Repressors Other Cell Cycle Proteins |
| E10f | bystin | Other Cell Adhesion Proteins |
| E10g | thrombospondin 2 (THBS2; TSP2) | Matrix Adhesion Receptors Cell-Cell Adhesion Receptors |
| E11a | transcription factor ETR101 | Basic Transcription Factors |
| E11c | Sp2 protein | CDK Inhibitors |
| E11e | tristetraproline (TTP); TIS11; ZFP36; growth factor-inducible nuclear protein 475 (NUP475) | Transcription Activators & Repressors |
| E12b | transcription factor IIIC box B-binding subunit | Cyclins |
| E12f | trophinin-associated protein (TROAP); tastin | Cell-Cell Adhesion Receptors |
| E12k | CC chemokine receptor type 2 (CMKBR2; CCCKR2; CCR2); monocyte chemoattractant protein 1 receptor B (MCP1RB) | Growth Factor & Chemokine Receptors Other Receptors (by Activities) |
| E13h | integrin beta 3 (ITGB3); platelet | Cell-Cell Adhesion Receptors |
| | membrane glycoprotein IIIA (GP3A); CD61 antigen | |
| E13m | microsomal stress 70 protein ATPase core | Xenobiotic Transporters |
| E13n | glutathione S-transferase theta 1 (GSTT1) | Xenobiotic Transporters Apoptosis-Associated Proteins |
| E14d | DNA-binding protein HIP116; ATPase; SNF2/SWI2-related protein | Basic Transcription Factors |
| E14i | integrin alpha L (ITGAL); lymphocyte function-associated 1 alpha subunit (LFA1A); CD11A antigen | Cell-Cell Adhesion Receptors |
| E14k | interleukin 7 receptor alpha subunit (IL7R-alpha; IL7RA); CDW127 antigen | Interleukin & Interferon ReceptorsCell Receptors (by Ligands) |
| | **BLOCK F** | |
| F01d | bone morphogenetic protein 4 (BMP4); BMP2B | Growth Factors, Cytokines & Chemokines |
| F01k | follistatin-like protein 1 (FSTL1); follistatin-related protein (FRP) | Other Extracellular Communication Proteins |
| F02c | alpha-1-acid glycoprotein 1 (AGP1);orosomucoid 1 (OMD1) | Other Extracellular Communication Proteins |
| F02f | vascular endothelial growth factor (VEGF); vascular permeability factor (VPF) | Growth Factors, Cytokines & Chemokines |
| F02i | alpha calcitonin | Hormones |
| F03f | pleiotrophin (PTN); osteoblast specific factor 1 (OSF1); heparin-binding neurite growth-promoting factor 1 (HBNF1; NEGF1) ; heparin-binding growth- associated molecule (HB-GAM); heparin-binding growth factor 8 (HBGF8) | Growth Factors, Cytokines & Chemokines |
| F03h | macrophage inflammatory protein 1 beta (MIP1-beta); T-cell activation protein 2 (AT2); PAT 744; H400; SIS-gamma; lymphocyte activation gene 1 protein (LAG 1); HC21; small inducible cytokine subfamily A member 4 (SCYA4); G 26 T-lymphocyte secreted protein | Growth Factors, Cytokines & Chemokines |
| F03j | interleukin 10 (IL10); cytokine synthesis inhibitory factor (CSIF) | Interleukins & Interferons |
| F03m | Matrix metalloproteinase 2 (MMP-2) | Metalloproteinases |
| F04b | 90-kDa heat-shock protein A (HSP90A); HSP86; HSPCA | Heat Shock Proteins |
| F04c | B94 protein | Other Extracellular Communication Proteins |
| F04j | interleukin 13 (IL13) | Interleukins & Interferons |
| F05n | Tissue inhibitor of metalloproteinases 1 (TIMP-1) | extracellular matrix proteins, protease inhibitors |
| F06a | glutathione peroxidase-gastrointestinal (GSHPX-GI); glutathione peroxidase-related protein 2 (GPRP) | Xenobiotic Transporters Apoptosis-Associated Proteins |
| F06e | endothelin 3 (EDN3; ET3) | Growth Factors, Cytokines & Chemokines |
| F06g | S100 calcium-binding protein A8 (S100A8); calgranulin A (CALA); migration inhibitory factor-related protein 8 (MRP8); leukocyte L1 complex light chain; cystic fibrosis antigen (CFAG) | Growth Factors, Cytokines & Chemokines |
| F06m | matrix metalloproteinase 8 (MMP8); neutrophil collagenase (CLG1); PMNL collagenase (PMNL-CL) | Metalloproteinases |
| F06n | tissue inhibitor of metalloproteinases 2 (TIMP-2) | extracellular matrix proteins, protease inhibitors |
| F07g | platelet-derived growth factor A subunit (PDGFA; PDGF1) | Growth Factors, Cytokines & Chemokines |
| F07j | interleukin 12 beta subunit (IL12-beta; IL12B); cytotoxic lymphocyte maturation factor 40-kDa subunit (CLMF p40); NK cell stimulatory factor subunit 2 (NKSF2) | Interleukins & Interferons |
| F07m | matrix metalloproteinase 9 (MMP9); gelatinase B; 92-kDa type IV collagenase (CLG4B) | Metalloproteinases |
| F10e | small inducible cytokine subfamily A member 5 (SCYA5); regulated on activation normal T-cell-expressed & secreted protein (RANTES); T-cell specific protein p288 (TCP288) | Growth Factors, Cytokines & Chemokines |
| F10j | interleukin 9 (IL9); T-cell growth factor p40 | Interleukins & Interferons |
| F11e | macrophage inflammatory protein 1 alpha (MIP1-alpha); tonsillar lymphocyte LD78 alpha protein; G0S19-1 protein; PAT 464.2; SIS-beta; small inducible cytokine subfamily A member 3 (SCYA3) | Growth Factors, Cytokines & Chemokines |
| F11k | insulin-degrading enzyme; insulysin; insulinase; insulin protease | Other Enzymes involved in Protein Turnover |
| F11m | matrix metalloproteinase 16 (MMP16); membrane-type matrix metalloproteinase 3 (MT-MMP3); MMP-X2 | Metalloproteinases |
| F13b | glutathione S-transferase mu 1 (GSTM1; GST1); HB subunit 4; GTH4 | Other Stress Response Proteins Other Apoptosis-Associated Proteins |
| F13d | epidermal growth factor (EGF); beta-urogastrone (URG) | Growth Factors, Cytokines & Chemokines |
| F13f | thymosin beta 10 (TMSB10; THYB10); PTMB10 | Growth Factors, Cytokines & Chemokines |
| F13j | thymosin beta 4 X chromosome (TMSB4X) | Other Extracellular Communication Proteins |

The differential expression of the identified genes was further verified on a newly recruited pool of non-diabetic controls (22 subjects) *versus* a newly recruited DM patients pool (38 subjects) in study part B (Main Study). In said study part B, expression of single genes was quantified and parallel ophthalmologic examinations were performed. All further experimental data discussed below is from said study part B.

Of the genes listed in Tab. 1, NF-kappa-B (D05m), XIAP (C04k), MMP-9 (F07m), MMP-2 (F03m), TIMP-1 (F05n) and TIMP-2 (F06n) were selected for further evaluation as potential molecular markers for DRP due to significant differences in their expression levels in diabetic patients in comparison to non-diabetic controls. In said further evaluation, MMP-2 and MMP-9 were evaluated by enzyme activity assays, the other potential molecular markers by measurement of their expression levels.

Furthermore, Ly-GDI has been identified as potential molecular marker for DRP due to its enhanced expression in diabetic patients (compare example 5, Fig. 2).

MMP-2 activity in serum was classified as follows (all values are given in relative units): negative (-; 0.00-0.34), enhanced (+; 0.35-0.79), highly increased (++; 0.80-1.29) and extremely increased (+++; higher than 1.29).

MMP-9 activity in serum was classified as follows (all values are given in relative units): negative (-; 0.00-0.49), enhanced (+; 0.50-0.89), highly increased (++; 0.90-1.44) and extremely increased (+++; higher than 1.44).

Enhanced and increased MMP-2 activities were found in the serum of 72 %, 67 %, and 63 % of patients with proliferative, highly proliferative and no active proliferative DRP, respectively (Example 7, Tab. 8). The MMP-2 activities were highly increased in 44 %, 44 %, and 19 % of patients with proliferative, highly proliferative and no active proliferative DRP. 62 % of smokers and 78 % of non-smokers demonstrated at least enhanced MMP-2 activities; exactly one half of these subjects in each group (31 % and 39 % respectively) revealed highly increased MMP-2 serum levels.

Enhanced and increased MMP-9 activities (Example 7, Tab. 8) were found in the serum of 72 % and 81 % of cases with and without proliferative DRP, respectively, and, with only one exception, in all patients of the subgroup with highly proliferative DRP. The MMP-9 activities were highly increased in 56 %, 67 %, and 31 % of patients with proliferative, highly proliferative and no active proliferative DRP. In the subgroups B and C (compare Tab. 3) these numbers were 80 %, 100 %, 40 % and 44 %, 63 %, 14 %, respectively. Almost an equal amount of smokers and non-smokers (85% and 83%, respectively) demonstrated at least enhanced MMP-9 activities; 38 % of smokers and 48 % of non-smokers demonstrated highly increased MMP-9 serum levels.

The correlation between DRP and the levels (MMP-2 and MMP-9: activity levels; all other molecular markers: expression levels) of the different potential molecular markers was statistically evaluated (example 8). Analysis of variance was done using ANOVA, pairwise testing was done by the Tukey test. The results are shown in Tab. 2.

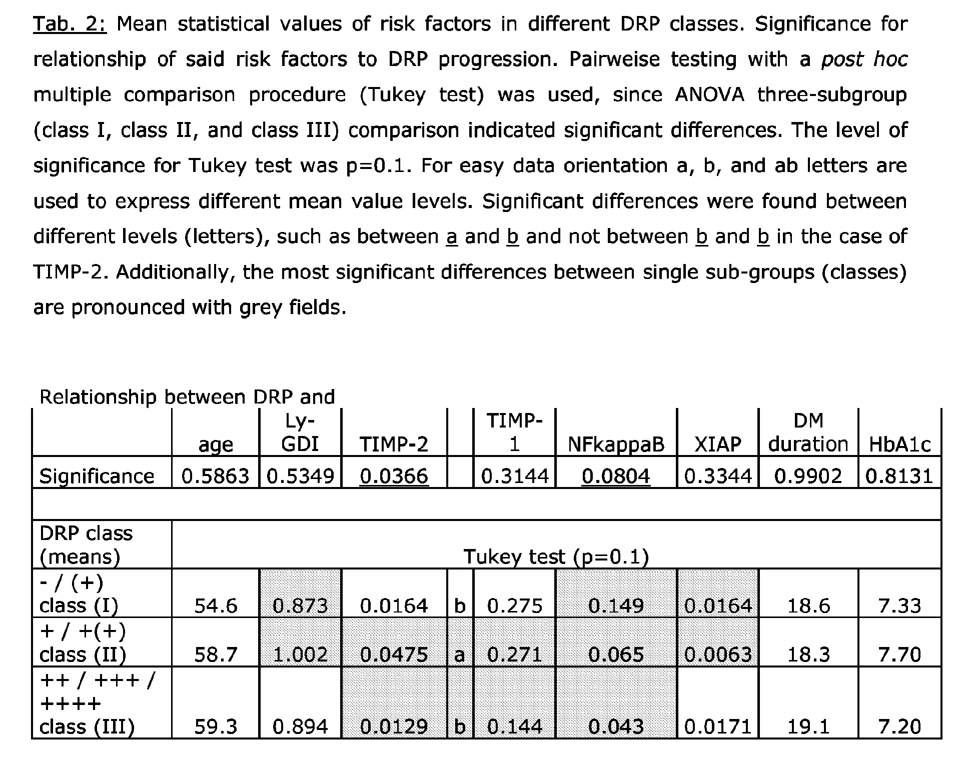

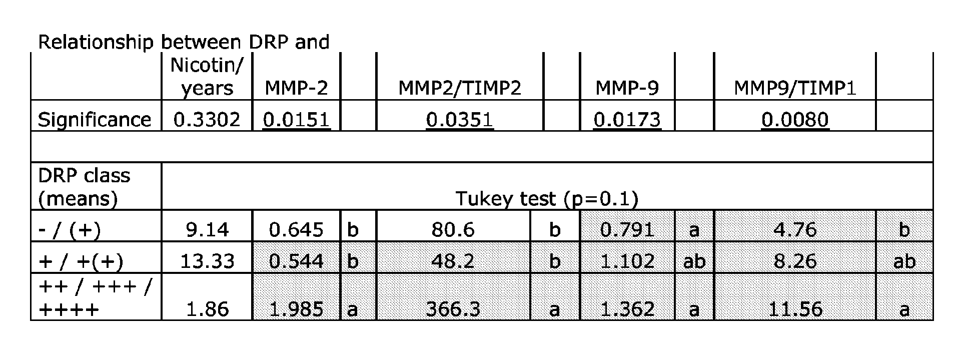

In the following, "increased/decreased value" means "increased/decreased in comparison to the mean values for the DRP classes listed in Tab. 2".

There are significant differences between the DRP classes for the following parameters: NFkappaB, TIMP-2, MMP-2, ratio MMP-2/TIMP-2, MMP-9 and ratio MMP-9/TIMP-1. Said significant differences are marked by underlined figures in Tab. 2. The increasing potential for DRP progression to the next higher level is marked in Tab. 2 by grey shading. Thus, increased values of Ly-GDI in DRP class I indicate a potential for progression into class II; decreased TIMP-2 values in class II indicate a potential for progression into class III, etc..

Taken together, statistically significant is the correlation between
(A) the potential DRP progression and increased values of MMP-9 and MMP-9/TIMP-1 and decreased values of NF-kappaB, in all DRP classes;
(B) the potential DRP progression of class I into class II and increased values of Ly-GDI as well as decreased values of XIAP; and
(C) the potential DRP progression of class II into class III and decreased values of TIMP-1, TIMP-2 values, which are at most 2 times the reference value but preferably decreased, as well as increased values of MMP-2 and MMP-2/TIMP-2.

The following parameters are therefore relevant for a prediction of a potential DRP progression between the three DRP classes:
Class I to Class II (altogether 5 parameters): Ly-GDI, NF-kappaB, XIAP, MMP-9, and MMP-9/TIMP-1; for sure prognosis at least the following 3 parameters should be measured: NF-kappaB, MMP-9, and MMP-9/TIMP-1.

Preferably, the single parameter values are considered as contributing to the risk for DRP progression, if
- Ly-GDI patient values (PV) are higher than the respective reference value: in our study those values PV > 0.85 relative units (later r.u.);
- NF-kappaB patient values are at least 1.5 times lower than the respective reference value: in our study those values PV < 0.14 r.u.;
- XIAP patient values are at least 1.5 times lower than the respective reference value: in our study those values PV < 0.013 r.u.;
- MMP-9 patient values are at least 1.3 times higher than the respective reference value: in our study those values PV > 0.9 r.u.; and/or
- MMP-9/TIMP-1 patient values are at least 1.5 times higher than the respective reference value: in our study those values PV > 5.0 r.u.

Class II to Class III (altogether 7 parameters): TIMP-2, TIMP-1, NF-kappaB, MMP-2, MMP2-/TIMP-2, MMP-9, and MMP-9/TIMP-1; for sure prognosis at least the following 5 parameters should be measured: TIMP-2, TIMP-1, MMP-2, MMP2-/TIMP-2, and MMP-9/TIMP-1.

Preferably, the single parameter values are considered as contributing to the the risk for DRP progression, if
- TIMP-2 patient values are not higher than 2 times compared to the respective reference value: in our study those values PV < 0.034 r.u. The lower the TIMP-2 level, the more probable is a Class II to Class III progression. Nevertheless, a slight increase in TIMP-2 up to 2 times in comparison to the control group is not lowering the risk of progression from Class II to Class III;
- TIMP-1 patient values are lower than 0.5 times of the respective reference value: in our study those values PV < 0.18 r.u.;
- NF-kappaB patient values are at least 3 times lower than the respective reference value: in our study those values PV < 0.065 r.u.;
- MMP-2 patient values are at least 1.5 times higher than the respective reference value: in our study those values PV > 0.9 r.u.;
- MMP2-/TIMP-2 patient values are at least 1.5 times higher than the respective reference value: in our study those values PV > 120 r.u.;
- MMP-9 patient values are at least 1.5 times higher than the respective reference value: in our study those values PV > 1.1 r.u.; and/or
- MMP-9/TIMP-1 patient values are at least 2.5 times higher than the respective reference value: in our study those values PV > 8.6 r.u.

In summary, the following 9 parameter values are considered as risk factors in the context of present invention: increased values of Ly-GDI, MMP-2, MMP-2/TIMP-2, MMP-9, MMP-9/TIMP-1; values of TIMP-2, which are not higher than two times the reference value, and which are preferably decreased; decreased values of TIMP-1, NF-kappaB, XIAP.

The Ly-GDI, MMP-2, MMP-2/TIMP-2, MMP-9, MMP-9/TIMP-1, TIMP-2, TiMP-1, NR-kappaB and XIAP values were determined in DM patients with different duration of DM and different levels of proliferative DRP. The results are summarized in Tab. 3.

**Tab. 3: Summarized information about the parameters and DRP risk factors evaluated in DM patients. The column "summarized points" indicates the grade of DRP risk calculated for individual patients. The final sum of points (maximum 9) is calculated by addition of individual points given for each of the 9 parameters (NFkappaB, XIAP, etc.): "-" parameter not measured"; "0" contribution of the parameter to the overall risk is minimal and therefore negligible for risk calculation (i.e. no risk factor); "1" contribution of the parameter is considerable and should be included in the overall risk calculation (i.e. risk factor).**

| No. | Age | sex | DM-Duration [years] | nicotine [years] | NF kappa B | XIAP | TIM P-1 | MM P-9 | MM P-9/TIMP1 | TIMP-2 | MMP-2 | MM P-2 /TI MP-2 | Ly-GDI | summaried pts. /maximum | Level of DRP proliferation |
|---|---|---|---|---|---|---|---|---|---|---|---|---|---|---|---|
| Group of DM-patients | | | | | | | | | | | | | | | |
| Subgroup A with DM-duration 3-9 years (10 people), 58.5±8.2 years old | | | | | | | | | | | | | | | |
| 34 | 59 | M | 3 | + 40 | - | - | 0 | 0 | 0 | - | 1 | 1 | - | 2/5 | no data |
| 56 | 65 | M | 3 | + 10 | 0 | 1 | 1 | 0 | 0 | 1 | 0 | 0 | 0 | 3/9 | - |
| 11 | 51 | M | 4 | +8 | 1 | - | 1 | 1 | 1 | - | 1 | 1 | - | 6/6 | ++ |
| 33 | 47 | F | 5 | - | 1 | 1 | 1 | 1 | 1 | 0 | 0 | 0 | 1 | 6/9 | - |
| 44 | 57 | M | 6 | + 40 | 0 | 0 | 0 | 1 | 0 | 0 | 1 | 1 | 0 | 3/9 | - |
| 16 | 68 | M | 6 | - | 1 | 1 | 1 | 0 | 0 | 0 | 0 | 0 | 1 | 4/9 | - |
| 17 | 66 | M | 7 | - | 1 | - | 1 | 0 | 1 | - | 0 | 0 | - | 3/6 | +++ |
| 55 | 49 | M | 8 | + 40 | 1 | - | 0 | 0 | 0 | - | 0 | 0 | - | 1/6 | +(+) |
| 37 | 54 | F | 8 | - | 1 | 1 | 0 | 1 | 0 | 1 | 1 | 1 | 1 | 7/9 | - |
| 6 | 69 | M | 9 | +40 | 1 | 0 | 1 | 1 | 1 | 0 | 0 | 0 | 1 | 5/9 | - |
| Highly increased risk of DRP/secondary diseases: 33% of patients | | | | | | | | | | | | | | | |

| Subgroup B with DM-duration 10-19 years (11 people), 59.4±5.9 years old | | | | | | | | | | | | | | | |
|---|---|---|---|---|---|---|---|---|---|---|---|---|---|---|---|
| 38 | 56 | M | 10 | + 4 | 1 | 1 | 1 | 1 | 1 | 0 | 1 | 0 | 1 | 7/9 | - |
| 24 | 63 | M | 12 | - | 1 | 1 | 0 | 1 | 0 | 0 | 0 | 0 | 1 | 4/9 | (+) |
| 20 | 65 | M | 12 | + 7 | 1 | 1 | 0 | 1 | 0 | 1 | 1 | 0 | 1 | 6/9 | - |
| 36 | 63 | M | 13 | - | 1 | - | 1 | 1 | 1 | - | 0 | 0 | - | 4/6 | + |
| 27 | 57 | F | 14 | - | 1 | - | 0 | 1 | 0 | - | 1 | 1 | - | 4/6 | + |
| 1 | 61 | M | 15 | - | 1 | 1 | 1 | - | - | 0 | - | - | 1 | 4/5 | - |
| 50 | 49 | F | 18 | + 35 | 1 | 1 | 1 | 0 | 0 | 0 | 1 | 1 | 1 | 6/9 | - |
| 41 | 53 | M | 18 | - | 1 | 1 | 1 | 1 | 1 | 1 | 1 | 0 | 0 | 7/9 | - |
| 46 | 67 | F | 18 | - | 1 | 1 | 1 | 0 | 0 | 0 | 0 | 1 | 0 | 4/9 | (+) |
| 26 | 54 | F | 19 | - | 1 | - | 1 | 1 | 1 | - | 1 | 1 | - | 6/6 | +(+) |
| 30 | 65 | M | 19 | + 18 | 0 | 1 | 0 | 1 | 0 | 1 | 1 | 0 | 1 | 5/9 | - |
| Highly increased risk of DRP/secondary diseases: 73% of patients | | | | | | | | | | | | | | | |

| Subgroup C with the DM-duration of DM more than 20 years (17 people), 60.7±12.8 years old | | | | | | | | | | | | | | | |
|---|---|---|---|---|---|---|---|---|---|---|---|---|---|---|---|
| 8 | 38 | F | 20 | + 5 | 1 | - | 1 | 1 | 1 | - | 0 | 0 | - | 4/6 | +++ |
| 32 | 62 | M | 20 | - | 1 | - | 1 | 1 | 1 | - | 1 | 1 | - | 6/6 | +++ + |
| 31 | 65 | F | 20 | - | 1 | 1 | 1 | 1 | 1 | 0 | 1 | 1 | 1 | 8/9 | (+) |
| 5 | 67 | F | 20 | - | 0 | - | 1 | 1 | 0 | - | 1 | 1 | - | 4/6 | ++ |
| 18 | 23 | F | 23 | - | 1 | 1 | 1 | 0 | 0 | 1 | 0 | 0 | 0 | 4/9 | - |
| 43 | 65 | M | 23 | - | 0 | 0 | 1 | 1 | 0 | 1 | 0 | 0 | 0 | 3/9 | - |
| 15 | 67 | M | 23 | - | 1 | 1 | 1 | 1 | 1 | 0 | 1 | 0 | 0 | 6/9 | (+) |
| 7 | 63 | M | 25 | - | 0 | - | 1 | 0 | 1 | - | 0 | 0 | - | 2/6 | + |
| 25 | 67 | M | 27 | - | 1 | 1 | 1 | 0 | 0 | 0 | 0 | 0 | 1 | 4/9 | - |
| 29 | 67 | M | 29 | - | 1 | - | 1 | 1 | 1 | - | 1 | 1 | - | 6/6 | No data |
| 2 | 65 | M | 30 | - | 1 | - | 1 | - | - | - | - | - | 1 | 3/3 | ++ |
| 13 | 66 | M | 31 | + 40 | 0 | - | 0 | 0 | 0 | - | 0 | 0 | - | 0/6 | +(+) |
| 28 | 46 | F | 33 | + 1 | 1 | 1 | 0 | 1 | 0 | 0 | 0 | 0 | 0 | 3/9 | - |
| 23 | 66 | F | 33 | - | 1 | - | 1 | 1 | 0 | - | 1 | 1 | - | 5/6 | ++ |
| 40 | 70 | M | 38 | - | 1 | 1 | 1 | 0 | 1 | 0 | 0 | 0 | 1 | 5/9 | - |
| 19 | 66 | F | 39 | - | 1 | 1 | 1 | 1 | 1 | 1 | 0 | 0 | 1 | 7/9 | - |
| 51 | 70 | F | 43 | - | 0 | 0 | 0 | 0 | 0 | 0 | 0 | 0 | 0 | 0/9 | - |
| Highly increased risk of DRP/secondary diseases: 59% of patients | | | | | | | | | | | | | | | |

The sum of risk factors (0: risk factor not present; 1: risk factor present) should be set in relation the amount of parameters that could be measured. For example, for the patients No. 34, No. 56 and No. 11 in Tab. 3 this would give the values 2/5 x 100 = 40%, 3/9 x 100 = 33%, and 6/6 x 100 = 100%, respectively. Resulting values equal or over 67 % (i.e. at least two thirds of the measured parameters are risk factors) indicate a highly increased risk of DRP development or progression; resulting values equal or below 33 % (i.e. one third or less of the measured parameters are risk factors) indicate a low risk of DRP development or progression. Patients with middle values - above 33 % and below 67 % - should be advised to repeat the test in a few months time in order to evaluate the trend of development.

The measurements of the activity in serum showed an increased or even highly increased level of both MMP-2 and MMP-9 enzymatic activity in the majority of DM-patients compared to non-diabetic controls. Particularly high levels were observed for MMP-9 activity, and those were increased in patients with proliferative DRP. With only one exception noted, the MMP-9 activity was increased in all patients with highly proliferative retinopathy. This correlation can be seen well in subgroups B and C with a longer DM-duration of 10-19 and 20-43 years respectively. Highly increased activities of both MMP-2 and MMP-9 were observed in more than twice as many cases with highly proliferative retinopathy compared to patients with no detectable active proliferation.

Moreover, and quite surprisingly, not the activity of MMP-2 and MMP-9 in blood serum/plasma alone, but the ratio of activity of said MMPs in blood serum/plasma to the expression of the corresponding tissue inhibitor of MMP (MMP-2/TIMP-2 and MMP-9/TIMP-1) in circulating leukocytes is an even more important - if not the most important - parameter for the risk evaluation. Furthermore, it is possible to differentiate risk factors between classes I, II and III: while an increasing ratio of MMP-9 to TIMP-1 is the risk factor for both class I and class II in the DRP progression to the next higher class, the ratio MMP-2 to TIMP-2 is only important for the prediction of the progression from class II to class III.

The calculated statistic to overall high risk in single diabetic sub-groups is well in agreement with current data published by other authors (Cheung, A.T. et al., Endocr. Pract. 7:358-363 (2001)). Moreover, roughly after the first decade of DM duration, indeed, the risk for secondary complications increases considerably, at least more than twofold. Contrary, in advanced age the potential risk is slightly decreased due to slowing of all metabolic processes generally.

Furthermore, present data indicates that the previously suggested prediction of the occurrence or the severity of DRP based on a single HbAlc value alone is impossible (compare Tab. 2). Said prediction was based on the positive correlation between individual HbAlc (long term blood glucose level) values and the status of proliferative retinopathy ( Lovestam-Adrian, M. et al., J. Diabetes Complications 15:287-294 (2001) ; Porta, M. et al., Diabetologia 44:2203-2209 (2001); Henricsson, M. et al., Diabetes Care 26:349-354 (2003)).

In case that only a limited number of parameters can and will be used for clinical evaluation of DRP progression, the ratio MMP-9/TIMP-1 should be calculated for all three classes of patients, whereas the ratio MMP-2/TIMP-2 should be calculated for patients of class II only.

The present invention is described in more detail by reference to the following examples. It should be understood that these examples are for illustrative purpose only and are not to be construed as limiting the invention.

### Examples

In the following examples, standard techniques of recombinant DNA technology were used that were described in various publications, e.g. Sambrook et al. (1989), Molecular Cloning: A Laboratory Manual, Cold Spring Harbor Laboratory, or Ausubel et al. (1987), Current Protocols in Molecular Biology 1987-1988, Wiley Interscience. Unless otherwise indicated, all devices, enzymes, reagents and kits were used according to the manufacturers' instructions.

### Example 1: Subjects

DM patients as well as non-diabetic controls were recruited for these investigations. The diagnosis of DM was based on a detailed clinical history and biochemical examination of the recruited persons. All investigations conformed to the principles outlined in the Declaration of Helsinki and were performed with permission of the local Ethics Committee.

Patients that came to the Department of Ophthalmology for evaluation of diabetic retinopathy and that were subsequently scheduled for fluorescein angiography were asked to participate. Informed consent was obtained, and multiple data regarding DM as well as details possibly affecting the patient's general health status were recorded. Before angiography, approximately 20 ml of full blood anti-coagulated with K₃EDTA were drawn and delivered immediately for molecular biological examination.

All patients and non-diabetic controls received a full eye exam, which included complex ophthalmologic, particularly angiographic investigations in terms of occurrence and severity grade of proliferative retinopathy.

### Pilot Study

Sub-group mRNA pools (DM-females; DM-males; control-females; control-males; compare Tab.4) from isolated peripheral leukocytes were used for the pilot study using the cDNA array whose results are listed in Tab. 1. Individual protein samples of the subjects were used for 2D-PAGE and subsequent comparative 2D-PAGE imaging.

**Tab. 4: information about the subjects in the pilot study. "n": Number of subjects**

| Group | n | Male/ Female [n] | Mean age [years] |
|---|---|---|---|
| DM-patients | 13 | 8/5 | 47.9 ± 4.9 |
| Non-diabetic individuals | 11 | 5/6 | 30.1 ± 9.3 |
| Males | 13 | 13/0 | 42.0 ± 9.2 |
| Females | 11 | 0/11 | 37.0 ± 13.1 |
| Total | 24 | 13/11 | 39.7 ± 11.5 |

### Main Study

Clinical data: Non-diabetic controls were on the average 49.1 years old. From 22 subjects no one demonstrated an proliferative retinopathy. DM-patients were on the average 54.6 (subgroup A), 58.7 (subgroup B), and 59.3 years (subgroup C) old. They formed subgroups with no ("-", "(+)"), middle ("proliferative") ("+","+(+)", and severe ("highly proliferative") ("++", "+++", "++++") DRP, respectively (Tab. 5). Of 38 patients, 35 subjects suffered from DM type 2, and 3 subjects from DM type 1. 76 % of all patients were insulin dependent. 34% were smokers. DM duration varied considerably from 3 to 43 years. Therefore, 3 subgroups A, B, and C with a DM-duration of 3-9, 10-19, and more than 20 years respectively, were formed. A proliferative retinopathy was found in exactly 50 % of all DM-patients (with a male to female ratio of about 1:1, the mean age being 60.7±7.9 years). There were 50, 45, and 56 % of patients with proliferative disease in subgroups A, B, and C respectively. The most prominent proliferations (noted as "++", "+++" and "++++"; class III) were observed in 28 % of all DM-patients, and in 33, 9, and 37.5 % of subgroups A, B, and C, respectively. Proliferative disease (class II), highly proliferative disease (class III) and no active proliferative disease (class I) was observed in 58, 25, and 42 % of non-smokers and in 42, 33, and 58 % of smokers, respectively. No correlation between individual HbAlc (an indicator for the long-term blood glucose level) values and the occurrence or a grade of proliferative retinopathy severity was found. The clinical data of all patients and of the members of the control group is summarized in Tab. 5.

**Tab. 5: Information about the patients, the control group and about the parameters measured. Patients are grouped according to the DM-duration. HbA1c shows long-term blood glucose levels (normal values are 5.0-6.9); APT indicates any type of antiproliferative therapy. VO: venous occlusion; FS: Fuch's spot; M: myopia; R: non-diabetic retinosis; MD: macular degeneration; T: trauma; EG: epiretinal gliosis.**

| Nr. | age | sex | DM Type | DM-Duration | HbA1c | Insulin dependence | APT | Nicotin/ years | Proliferation |
|---|---|---|---|---|---|---|---|---|---|
| Subgroup A: DM-duration 3-9 years (n=10), 58.5±8.2 years old | | | | | | | | | |
| 34 | 59 | M | DM 2 | 3 | 11.3 | - | + | + 40 | No data |
| 56 | 65 | M | DM 2 | 3 | 7.5 | - | - | + 10 | - |
| 11 | 51 | M | DM 2 | 4 | 6.8 | - | + | + 8 | ++ |
| 33 | 47 | F | DM 2 | 5 | 8.5 | + | - | - | - |
| 44 | 57 | M | DM 2 | 6 | 8.2 | - | - | + 40 | (+) |
| 16 | 68 | M | DM 2 | 6 | 5.3 | + | + | - | (+) |
| 17 | 66 | M | DM 2 | 7 | 6.5 | + | + | - | +++ |
| 55 | 49 | M | DM 2 | 8 | 6.4 | + | + | + 40 | +(+) |
| 37 | 54 | F | DM 2 | 8 | 6.0 | - | - | - | - |
| 6 | 69 | M | DM 2 | 9 | 8.7 | - | - | + 40 | - |

| Subgroup B: DM-duration 10-19 years (n=11), 59.4±5.9 years old | | | | | | | | | |
|---|---|---|---|---|---|---|---|---|---|
| 38 | 56 | M | DM 2 | 10 | 7.0 | + | - | + 4 | - |
| 24 | 63 | M | DM 2 | 12 | 6.5 | + | - | - | (+) |
| 20 | 65 | M | DM 2 | 12 | 6.2 | - | - | + 7 | - |
| 36 | 63 | M | DM 2 | 13 | 7.3 | + | + | - | + |
| 27 | 57 | F | DM 2 | 14 | 7.7 | + | + | - | + |
| 1 | 61 | M | DM 2 | 15 | 9.0 | + | - | - | - |
| 50 | 49 | F | DM 1 | 18 | 5.3 | + | - | + 35 | - |
| 41 | 53 | M | DM 2 | 18 | 7.7 | + | + | - | - |
| 46 | 67 | F | DM 2 | 18 | 11.0 | + | + | - | (+) |
| 26 | 54 | F | DM 2 | 19 | 10.7 | + | + | - | +(+) |
| 30 | 65 | M | DM 2 | 19 | 6.1 | + | - | + 18 | - |

| Subgroup C: DM-duration more than 20 years (n=17), 60.7±12.8 years old | | | | | | | | | |
|---|---|---|---|---|---|---|---|---|---|
| 8 | 38 | F | DM 2 | 20 | 8.6 | + | + | + 5 | +++ |
| 32 | 62 | M | DM 2 | 20 | 9.1 | + | + | - | ++++ |
| 31 | 65 | F | DM 2 | 20 | 10.5 | + | - | - | (+) |
| 5 | 67 | F | DM 2 | 20 | 6.3 | + | + | - | ++ |
| 18 | 23 | F | DM 2 | 23 | 6.1 | + | + | - | - |
| 43 | 65 | M | DM 2 | 23 | 6.3 | + | + | No data | - |
| 15 | 67 | M | DM 2 | 23 | 5.8 | + | + | - | (+) |
| 7 | 63 | M | DM 2 | 25 | 7.7 | + | - | - | + |
| 25 | 67 | M | DM 2 | 27 | 8.1 | + | + | - | - |
| 29 | 67 | M | DM 2 | 29 | No data | + | + | - | No data |
| 2 | 65 | M | DM 2 | 30 | 6.6 | + | + | - | ++ |
| 13 | 66 | M | DM 2 | 31 | 6.4 | - | + | +40-50 | +(+) |
| 28 | 46 | F | DM 1 | 33 | 6.8 | + | + | + 1 | - |
| 23 | 66 | F | DM 2 | 33 | 6.5 | - | + | - | ++ |
| 40 | 70 | M | DM 1 | 38 | 7.0 | + | - | - | - |
| 19 | 66 | F | DM 2 | 39 | 7.2 | + | + | - | - |
| 51 | 70 | F | DM 2 | 43 | 7.8 | + | + | - | - |
| Group of non-diabetic controls | | | | | | | | | |

| Nr. | age | sex | Diagnosis | Nicotine/ Years | Proliferation |
|---|---|---|---|---|---|
| 3 | 69 | F | VO | - | (+) |
| 4 | 66 | F | VO | - | - |
| 9 | 29 | M | R | +10 | - |
| 10 | 45 | M | VO | +20 | - |
| 12 | 55 | M | R | - | - |
| 14 | 69 | F | VO | +50 | - |
| 21 | 27 | M | R | - | - |
| 22 | 65 | M | MD | +45 | - |
| 35 | 36 | M | R | +20 | - |
| 39 | 54 | F | FS | +30 | - |
| 42 | 23 | F | FS | - | - |
| 45 | 52 | F | R | +5 | - |
| 47 | 38 | M | R | - | - |
| 48 | 36 | F | R | +23 | - |
| 49 | 39 | F | M | +10 | - |
| 52 | 61 | F | EG | +7 | - |
| 53 | 52 | F | MD | - | - |
| 54 | 63 | M | EG | +2 | - |
| 57 | 67 | M | EG | - | - |
| 58 | 25 | M | T | - | - |
| 59 | 62 | M | VO | - | - |
| 60 | 48 | F | R | +25 | - |

### Example 2: Isolation of leukocytes

Blood samples (20 ml) anti-coagulated with heparin were collected from patients and controls. Leukocytes (including monocytes and stem cells) were separated using Ficoll-Histopaque gradients (Histopaque 1077, Sigma, Switzerland) as described previously (Golubnitschaja-Labudova, O. et al., Curr. Eye Res. 21:867-876 (2000)). Briefly, blood samples were diluted with an equal volume of physiological buffer solution (PBS, GibcoBRL, Switzerland). Then, 2 ml of histopaque were placed into 10 ml sterile centrifuge tubes and 5 ml of diluted blood samples were carefully layered onto each histopaque gradient. Gradients were centrifuged at 475 g and 20 °C for 15 min. The leukocyte bands were removed from the interface between the plasma and histopaque layers of each tube and collected into one 50 ml tube. The total volume was brought to 50 ml with cold Dulbecco's Modified Eagle Medium (DMEM, GibcoBRL, Switzerland). The cell suspension was washed three times with DMEM and the total number of cells was determined. Cells were finally suspended in PBS and aliquoted into Eppendorf tubes at 10⁷ cells/tube. After centrifugation cell pellets were dried and stored at -70 °C until either mRNA or protein isolation.

### Example 3: Isolation of total RNA, mRNA and first-strand-cDNA synthesis

Isolation of total RNA from aliquoted samples of isolated leukocytes (Example 2) and the concomitant mRNA isolation were performed using RNAzol™ B (WAK-Chemie Medical GmbH, Germany) and Oligotex mRNA Mini Kit (Qiagen, Germany), respectively, according to the protocols supplied by the manufacturers. cDNA synthesis was performed using the "First-Strand cDNA Synthesis Kit" (Amersham Biosciences, UK). For each cDNA synthesis, 1 µg mRNA was reverse transcribed using an oligo(dT)₁₈ primer in a final volume of 15 µl each, according to the protocol supplied by the manufacturer.

### Example 4: Hybridization to DNA Array

A) Preparation of biotin-labeled cDNA probes: 100 ng of cDNA (Example 3) were labeled using a SpotLight™ Random Primer Labeling Kit (BD Biosciences Clontech, USA). Template cDNA together with 5 µl of 10x Random Primer Mix were heated to 97 °C for 3 min in a final volume of 31 µl and chilled quickly on ice. After adding the reaction mix (5 µl 10x Klenow Reaction Buffer, 5 µl 10x Klenow Labeling Mix, 1 µl Klenow Enzyme and 8 µl ddH₂O) the labeling reaction was performed at 37 °C for 30 min. Then the reaction was stopped by adding 2 µl of 0.5 M EDTA (pH 8.0).
B) Purification of biotin-labeled probe: Unincorporated biotin-labeled nucleotides and small (<0.1 kb) cDNA fragments were removed using the NucleoSpin Extraction columns according to the protocol supplied by the manufacturer (BD Biosciences Clontech, USA). The concentration of the newly synthesized biotin-labeled probes was determined by UV spectroscopy. Finally, the biotin-labeled probes were stored at -20 °C until hybridization to an Atlas Array.
C) Hybridization of biotin-labeled probes to Atlas Array: For the hybridization, an Atlas^{™} Human Cardiovascular Array (Cat. #7734-1, BD Biosciences Clontech, USA) with altogether 588 cardiovascular diseases relevant genes, as well as a SpotLight™ Chemiluminescent Hybridization & Detection kit (Clontech) were used. Each Atlas Array (membrane) was wetted by placing it in a dish of deionized H₂O, transferred to the hybridization bottle and pre-hybridized in the hybridization mix at 42 °C for 3 h before the hybridization. Each hybridization was performed overnight at 42 °C with individual biotinylated cDNA probes. These probes were denatured in 100 mM NaOH at 68 °C for 20 min and neutralized with 0.5 M NaH₂PO₄ (pH 7.0) at 68 °C for 10 min before the hybridization in the Hybridizer (Techne, UK).
D) Stringency Washes: Hybridization solution was discarded and the membranes were washed 4 times in 200 ml of Wash solution 1 (2xSSC and 1 % SDS) for 30 min at 60 °C with continuous agitation. Wash solution 1 was then replaced with Wash solution 2 (0.1xSSC and 0.5 % SDS), and the membranes were washed two more times for 30 min at 48 °C.
E) Probe detection and signal visualization: The membranes were incubated first in 25 ml Blocking Buffer per Atlas Array at room temperature for 1 hour before an incubation with Streptavidin-HRP Conjugate (final dilution of 1:300) for 1 hour with constant gentle agitation. Afterwards the membranes were washed 4 times in 1x Wash Buffer (SpotLight™ Chemiluminescent Hybridization & Detection kit, BD Biosciences Clontech) for 10 min with concomitant equilibration in Substrate Equilibration Buffer (kit) for 5 min at room temperature before the incubation in 8 ml Working Solution (the mix of Luminol/Enhancer Solution and Stable Peroxide Solution, supplied in the kit) per membrane for 5 min. After removing excess liquid the Atlas Arrays were processed by autoradiography with exposure times of 1, 2, 5, 10 and 30 min. The exposed spots were scanned and analyzed with AtlasImage software (BD Biosciences Clontech). A resulting radiograph (exposure time 5 min) is shown in Fig. 1.

### Example 5: 2D-Page, identification and quantification of Ly-GDI

Ly-GDI was identified as potentially relevant for DRP using comparative 2D-PAGE imaging combined with MALDI-TOF protein sequence analysis followed by Western-blot quantification. 2D-PAGE images showing differential expression of Ly-GDI in four sub-groups (DM-females, DM-males; control-females, control-males) of the pilot study (Tab. 4) are given in Fig. 2.

In more detail, comparative 2D-PAGE imaging followed by tandem mass spectrometry analysis was performed as describe below. The identified differentially expressed Ly-GDI protein was further quantified in individual samples using "Western-blot" analysis as described below.
A) Two-dimensional PAGE (2D-PAGE): A 400 µg aliquot of each protein sample isolated from circulating leukocytes (example 2) of either individual patients or controls was utilized for 2D-PAGE analysis performed two times for each sample. First-dimensional separation was performed in immobilized pH gradient (IPG) strips (Bio-Rad) in the range of pH 4-7 following the manufacturer's protocol. 125 µl protein samples containing rehydration buffer (8 M urea, 10 mM DTT, 1 % CHAPS, 0.25 % Bio-Lyte, pH 4-7) was loaded on the IPG-strips and subjected to 14 kVh overnight at 20 °C in a PROTEAN IEF Cell. Following first-dimensional separation, the extruded IPGstrips were equilibrated in gel equilibration buffer I (50 mM Tris-HCl, 6 M urea, 30 % glycerol, 2 % SDS, 1 % DTT), followed by equilibration in buffer II (50 mM Tris-HCl, 6 M urea, 30 % glycerol, 2 % SDS and 260 mM iodacetamide) for 10 min before loading them onto polyacrylamide gels (12 % SDS-PAGE) for the second-dimensional resolution in Mini-PROTEAN 3 (Bio-Rad). For size determination of the separated proteins, colored protein standards (Cat. Nr. 161-0318, Bio-Rad) were loaded onto the gels. After electrophoresis, the separated proteins were visualized using silver staining which provides a high sensitivity for protein expression analysis. Differential gene expression was analyzed using specific software (Bio-Rad). Differentially expressed protein spots were excised from the gels and analysed by tandem mass spectrometry.
B) Tandem mass spectrometry: Non-separated peptide mixtures from the tryptic, in-gel digest of unknown proteins from the 2D-PAGE were analyzed by nanoelectrospray tandem mass spectrometry. The tryptised peptides from the wells of the microtiter plates were desalted and concentrated on a pulled capillary containing approximately 100 nl of POROS R2 reverse phase material (Perseptive Biosystems, Framingham, MA) and eluted with 1 ml of 60 % methanol in 5 % formic acid directly into the nanoelectrospray needle. Electrospray mass spectra were acquired on a QSTAR Pulsar I quadrupole TOF tandem mass spectrometer (AB/MDS-Sciex, Toronto, Canada) equipped with a nanoelectrospray ion source (MDS Protana, Odense, Denmark). Fragmentation by tandem mass spectrometry yielded a stretch of sequence together with its location in the peptide (peptide sequence tag). With these sequence tags appropriate databases were searched.
C) Western-Blot analysis: All analyses were performed two times for each sample. Leukocytes were lysed by homogenisation in lysis buffer (9 M urea (Merck, Germany), 1 % DTT (Sigma, USA), 2 % CHAPS (Merck, Germany), 0.8 % Bio-Lyte, pH 3-10 (Bio-Rad, USA), 5 mM Pefabloc^{®} (Roche, Switzerland)), followed by a centrifugation step. The protein concentration was quantified by the DC-Protein Assay (Bio-Rad, USA). 40 µg protein of each sample were loaded onto 12 % SDS-polyacrylamide gels and electrophoresed to separate proteins. The proteins were then transferred to nitrocellulose membranes (Hybond ECL, Amersham Biosciences, UK) and afterwards incubated at room temperature in blocking-buffer (58 mM Na₂HPO₄, 17 mM NaH₂PO₄, 68 mM NaCl, 5 % nonfat dry milk powder; 0.1 % Tween 20^{®}) for 1 h. Primary antibody incubation was performed at room temperature using a 1:250 dilution of Ly-GDI C-20 sc-6047 antibodies (goat polyclonal IgG, 200 pg/ml; Santa Cruz, USA) in washing buffer I (58 mM Na₂HPO₄, 17 mM NaH₂PO₄, 68 mM NaCl, 1 % non-fat dry milk powder, 0.1 % Tween 20^{®}) for 1 h. The membranes were then washed four times in the same solution. The horseradish peroxidase-labelled anti-goat secondary antibody was incubated 1 h at room temperature with the membranes in washing buffer I, followed by three washes in washing buffer II (58 mM Na₂HPO₄, 17 mM NaH₂PO₄, 68 mM NaCl, 1 % nonfat dry milk powder; 0.3 % Tween 20^{®}) and three washes in washing buffer I. Then the membranes were reacted with chemiluminescent reagent ECL plus (Detection Kit, Amersham Biosciences, UK) for 1 h and processed for auto-radiography. The individual signals were measured densitometrically using the "Quantity One" imaging system (Bio-Rad, USA). LY-GDI protein expression rates of individual subjects in leukocytes quantified by Western blot are shown in Tab. 6.

**Tab. 6: Quantification (in relative units) of Ly-GDI expression in leukocytes. Control Numbers 61 to 67: additional control subjects.**

| Patient Number | Value of expression |
|---|---|
| 1 | 1.352 |
| 2 | 1.1 |
| 3 | 1.224 |
| 5 | 0.966 |
| 6 | 1.014 |
| 7 | 1.062 |
| 8 | 0.962 |
| 11 | 1.029 |
| 13 | 1.137 |
| 15 | 0.838 |
| 16 | 1.07 |
| 17 | 0.472 |
| 18 | 0.762 |
| 19 | 1.031 |
| 20 | 1.145 |
| 23 | 0.7 |
| 24 | 0.933 |
| 25 | 0.906 |
| 26 | 1.08 |
| 27 | 1.15 |
| 28 | 0.871 |
| 29 | 1.332 |
| 30 | 1.059 |
| 31 | 0.91 |
| 32 | 1.029 |
| 33 | 1.020 |
| 34 | 1.2 |
| 36 | 1.25 |
| 37 | 0.993 |
| 38 | 1.070 |
| 40 | 1.065 |
| 41 | 0.851 |
| 43 | 0.791 |
| 44 | 0.625 |
| 46 | 0.777 |
| 50 | 0.904 |
| 51 | 0.849 |
| 55 | 0.332 |
| 56 | 0.466 |

| Control Number | Value of expression |
|---|---|
| 3 | 1.224 |
| 4 | 0.999 |
| 9 | 0.652 |
| 10 | 1.231 |
| 12 | 1.000 |
| 14 | 0.851 |
| 21 | 1.003 |
| 22 | 0.99 |
| 35 | 1.277 |
| 39 | 1.115 |
| 42 | 0.936 |
| 45 | 0.62 |
| 47 | 0.661 |
| 48 | 0.682 |
| 49 | 0.852 |
| 52 | 0.732 |
| 53 | 0.401 |
| 54 | 0.212 |
| 57 | 0.648 |
| 58 | 1.211 |
| 59 | 0.452 |
| 60 | 0.237 |
| 61 | 0.629 |
| 62 | 0.827 |
| 63 | 0.578 |
| 64 | 0.676 |
| 65 | 0.689 |
| 66 | 0.863 |
| 67 | 0.795 |

### Example 6: Reverse Transcriptase PCR (RT-PCR) and Real-Time Quantitative PCR (RT-QPCR)

In order to detect the expression of the target genes in leukocytes and to optimize the reaction conditions for Real-Time Quantitative PCR, reverse transcriptase PCR was performed using specific primer sets designed to the house-keeping gene β-actin and the target genes. The primer sets were as follows:
β-actin:
   Forward: 5'GATGGTGGGCATGGGTCAG 3' (SEQ ID NO:17)
   Reverse: 5'TGGGGTTCAGGGGGGCCT 3' (SEQ ID NO:18)
NF-kappaB:
   Forward: 5'CTACAACCCAGGTCTGGAT 3' (SEQ ID NO:19)
   Reverse: 5'GTCACTGTGTGTGTTACCAGG 3' (SEQ ID NO:20)
XIAP:
   Forward: 5'GAAGATGACTTTTAACAGTTTTGAAG 3' (SEQ ID NO:21)
   Reverse: 5'AATTTGGGGATACTTTCCTGTG 3' (SEQ ID NO:22)
TIMP-1:
   Forward: 5'GGCCCCCTTTGAGCCC 3' (SEQ ID NO:23)
   Reverse: 5'CTCAGGCTGTTCCAGGGA 3' (SEQ ID NO:24)
TIMP-2:
   Forward: 5'TCACCCTCTGTGACTTCATC 3' (SEQ ID NO:25)
   Reverse: 5'GTCGAGAAACTCCTGCTTG 3' (SEQ ID NO:26)

cDNA synthesis was performed using a "First-Strand cDNA Synthesis Kit" (Amersham Biosciences, UK). The PCR mixture contained 1xPCR buffer (16.6 mM ammonium sulfate, 67 mM Tris, pH 8.8, 6.7 mM MgCl₂, 10 mM 2-mercaptoethanol), dNTPs (each at 1.25 mM), primer pairs (100 pM each per reaction), and 10 ng of cDNA-template in a final volume of 50 µl. Reactions were hot-started at 95 °C for 5 min before adding 1.5 units of Taq Polymerase (Red-Hot^{®}, ABgene, UK) at the annealing temperature of 56 °C followed by polymerization at 72 °C for 1 min. Amplification was carried out in a Perkin Elmer DNA Thermal Cycler TC480 for 45 cycles (penetration for 45 s at 95 °C, annealing for 45 sec at 56 °C, and polymerization at 72 °C for 30 s), followed by a final 7 min extension at 72 °C. Negative controls without DNA as well as positive controls with a sequenced template were performed for each set of PCR experiments. PCR products (50 µl) were directly loaded onto 3 % agarose gels ("Wide Range"-Agarose for analysis of DNA fragments longer than 50 bp, Sigma), stained with ethidium bromide after electrophoresis, directly visualized under UV illumination, and imaged using a specialized imaging system (MWG-Biotech, Germany). The specificity of each PCR amplification was controlled using the site specific restriction analysis of target PCR products. The amplification products underwent an extraction from the agarose gel using a DNA isolation kit (DNAClean^{™}, Hybaid-AGS GmbH, Germany) before digestion. They were digested in a final volume of 50 µl with 20 units of each restriction endonuclease for 4 h, according to conditions specified by the manufacturer (Roche, Switzerland), and imaged after electrophoresis (MWG-Biotech, Germany).

Results: RT-PCR of the house keeping gene, β-actin, and all target genes in the cDNA samples demonstrated the specific amplification of the target PCR products with the expected DNA fragment lengths. Further restriction analysis carried out on the isolated PCR products confirmed the expected sequences.

In order to profile precisely the changes in expression of target genes, RT-QPCR was used. SYBR^{®} Green I (Molecular Probes, USA) was utilized as the intercalation dye and fluorescent reporter molecule detecting the accumulation of the amplified double-stranded product in the iCycler iQIM Detection System (Bio-Rad, USA) according to the protocol supplied by the manufacturer. 50 ng of the cDNAs synthesized by above RT-PCR were used for each real-time PCR analysis. The reaction mixtures had the same contents as for RT-PCR with an exception of Red-Hot^{®} polymerase (ABgene, UK), which was substituted for Taq DNA polymerase (Roche, Switzerland) in order to avoid color signal disturbances. The same amplification program was used in both qualitative RT-PCR and quantitative real-time PCR analysis. The algorithm of the iCycler iQIM Detection System normalizes the reporter signal (non-intercalated SYBR^{®} Green I) to a passive reference and multiplies the standard deviation (SD) of the background signal in the first few cycles by a default factor of 10, to determine a threshold. The cycle at which this baseline level is exceeded is defined as threshold cycle (Cₜ). Cₜ depends on the initial template copy number and is proportional to the log of the starting amount of nucleic acid (Heid, C.A. et al., Genome Res. 6:986-994 (1996)). By subtracting the Cₜ values of the target genes from those of the housekeeping gene (β-actin), the data were normalized. The relative levels were calculated for each sample based on the differences in Cₜ values. The results of the quantification of the specific target gene transcription rates in leukocytes for NF-kappaB, XIAP, TIMP-1 and TIMP-2 are shown in Tab. 7. The mean values and statistical significance are listed in Tab. 2.

**Tab. 7: Gene transcription rates (normalized to β-actin)**

| No. patient | NF-kappaB | XIAP | TIMP-1 | TIMP-2 |
|---|---|---|---|---|
| 1 | 0.1250 | 0.00837 | 0.102 | 0.009 |
| 2 | 0.0625 | 0.01408 | 0.241 | 0.016 |
| 5 | 0.0769 | 0.01992 | 0.159 | 0.004 |
| 6 | 0.0647 | 0.01563 | 0.056 | 0.016 |
| 7 | 0.1487 | 0.00867 | 0.063 | 0.008 |
| 8 | 0.0242 | 0.02720 | 0.134 | 0.016 |
| 11 | 0.0490 | 0.00809 | 0.099 | 0.007 |
| 13 | 0.0797 | 0.01184 | 0.406 | 0.210 |
| 15 | 0.0282 | 0.00032 | 0.056 | 0.012 |
| 16 | 0.0156 | 0.00138 | 0.125 | 0.008 |
| 17 | 0.0136 | 0.00120 | 0.085 | 0.016 |
| 18 | 0.0372 | 0.00352 | 0.203 | 0.023 |
| 19 | 0.0385 | 0.00929 | 0.121 | 0.025 |
| 20 | 0.0335 | 0.00962 | 0.277 | 0.024 |
| 23 | 0.0206 | 0.00498 | 0.200 | 0.015 |
| 24 | 0.0442 | 0.00515 | 0.366 | 0.013 |
| 25 | 0.0398 | 0.00962 | 0.134 | 0.017 |
| 26 | 0.0508 | 0.00613 | 0.092 | 0.006 |
| 27 | 0.0263 | 0.00572 | 0.277 | 0.014 |
| 28 | 0.0474 | 0.01031 | 0.308 | 0.012 |
| 29 | 0.0717 | 0.02288 | 0.109 | 0.007 |
| 30 | 0.1830 | 0.07966 | 0.330 | 0.026 |
| 31 | 0.0544 | 0.02288 | 0.183 | 0.008 |
| 32 | 0.0563 | 0.04419 | 0.092 | 0.016 |
| 33 | 0.0583 | 0.01314 | 0.105 | 0.009 |
| 34 | 0.0625 | 0.00755 | 0.392 | 0.010 |
| 36 | 0.0335 | 0.00258 | 0.149 | 0.032 |
| 37 | 0.0670 | 0.00195 | 0.319 | 0.018 |
| 38 | 0.0544 | 0.00202 | 0.121 | 0.015 |
| 40 | 0.0237 | 0.00129 | 0.105 | 0.014 |
| 41 | 0.0947 | 0.00755 | 0.129 | 0.021 |
| 43 | 0.2176 | 0.01992 | 0.189 | 0.032 |
| 44 | 0.2665 | 0.05256 | 0.342 | 0.014 |
| 46 | 0.0854 | 0.01360 | 0.113 | 0.006 |
| 50 | 0.0769 | 0.00352 | 0.189 | 0.001 |
| 51 | 0.3139 | 0.02816 | 0.683 | 0.010 |
| 55 | 0.05 | 0.00292 | 0.637 | 0.015 |
| 56 | 0.1895 | 0.00898 | 0.139 | 0.019 |

| No. of control | | | | |
|---|---|---|---|---|
| 3 | 0.0372 | 0.01184 | 0.134 | 0.008 |
| 4 | 0.0981 | 0.02452 | 0.080 | 0.008 |
| 9 | 0.1387 | 0.01618 | 0.342 | 0.011 |
| 10 | 0.1051 | 0.00996 | 0.095 | 0.009 |
| 12 | 0.4061 | 0.00962 | 0.082 | 0.009 |
| 14 | 0.0694 | 0.01509 | 0.287 | 0.102 |
| 21 | 0.0213 | 0.00755 | 0.171 | 0.036 |
| 22 | 0.0213 | 0.00929 | 0.500 | 0.033 |
| 35 | 0.0237 | 0.00101 | 0.451 | 0.017 |
| 39 | 0.0442 | 0.00352 | 0.189 | 0.016 |
| 42 | 0.2031 | 0.01795 | 0.660 | 0.015 |
| 45 | 0.2415 | 0.02452 | 0.268 | 0.006 |
| 47 | 0.0797 | 0.00755 | 0.109 | 0.005 |
| 48 | 0.7320 | 0.05255 | 0.225 | 0.015 |
| 49 | 0.1830 | 0.00755 | 0.210 | 0.019 |
| 52 | 0.4353 | 0.00534 | 0.177 | 0.007 |
| 53 | 0.29282 | 0.01675 | 0.203 | 0.002 |
| 54 | 0.3078 | 0.02916 | 0.574 | 0.028 |
| 57 | 0.15157 | 0.02102 | 0.785 | 0.008 |
| 58 | 0.4353 | 0.06037 | 0.354 | 0.009 |
| 59 | 0.27321 | 0.03536 | 1.072 | 0.011 |
| 60 | 0.22974 | 0.02176 | 0.732 | 0.010 |

### Example 7: Zymography

For determination of gelatinase activity of MMP-2 (gelatinase A) and MMP-9 (gelatinase B) in blood serum "Ready-Gelatin-Gels" (Bio-Rad, USA) were used according to the instructions of the manufacturer. Two micro liters from individual serum samples were electrophoresed under non-reducing conditions using Criterion^{™} Precast Gel System (Bio-Rad, USA). After electrophoresis, each gel was incubated at room temperature in 2 % Triton X-100^{®} for 2 x 30 min in order to remove the traces of sodium dodecyl sulphate, and then incubated overnight at 37 °C in buffer (150 mM NaCl, 50 mM Tris-HCl, pH 7.6, containing 5 mM CaCl₂ and 0.02 % NaN₃). Afterwards a staining with 0.5 % Coomassie blue G-250 (Sigma, USA) was performed for each gel. The proteolytic activity of each gelatinase was identified as a clear band on a blue background according to the correspondent molecular weight of each gelatinase. Gels were dried between cellophane sheets with a GelAir™ Drying System (Bio-Rad, USA) and then scanned with a yellow filter using Adobe Photoshop (Adobe System, USA) in grey-scale mode. Densitometric analysis of zymographic lysis zones at 66 and 86 kDa for gelatinases A and B, respectively, was performed using "Quantity One" imaging system (Bio-Rad, USA). The zymography results are show in Tab. 8.

**Tab. 8: Results of densitometric quantification (in relative units) of the activity of MMP-2 and MMP-9 in blood serum. Column 3: expression of TIMP-2 in leukocytes; column 4: ratio MMP-2/TIMP-2; column 6: expression of TIMP-1 in leukocytes; column 7: ratio MMP-9/TIMP-1.**

| No of patient | Activity of MMP-2 | Expression of TIMP-2 | Ratio MMP-2 / TIMP-2 | Activity of MMP-9 | Expression of TIMP-1 | Ratio MMP-9 / TIMP-1 |
|---|---|---|---|---|---|---|
| 1 | No data | 0.009 | No data | No data | 0.102 | No data |
| 2 | No data | 0.016 | No data | No data | 0.241 | No data |
| 5 | 7.082 | 0.004 | 1770.5 | 1.109 | 0.159 | 6.93 |
| 6 | 0.245 | 0.016 | 15.31 | 1.257 | 0.056 | 22.45 |
| 7 | 0.224 | 0.008 | 28.00 | 0.619 | 0.063 | 9.90 |
| 8 | 0.082 | 0.016 | 5.13 | 1.528 | 0.134 | 11.40 |
| 11 | 1.500 | 0.007 | 214.29 | 1.370 | 0.099 | 13.98 |
| 13 | 0.221 | 0.210 | 1.05 | 0.744 | 0.406 | 1.83 |
| 15 | 0.894 | 0.012 | 74.5 | 0.837 | 0.056 | 14.95 |
| 16 | 0.499 | 0.008 | 62.38 | 0.138 | 0.125 | 1.10 |
| 17 | 0.519 | 0.016 | 32.44 | 0.744 | 0.085 | 8.75 |
| 18 | 0.488 | 0.023 | 21.22 | 0.180 | 0.203 | 0.89 |
| 19 | 0.421 | 0.025 | 16.84 | 0.956 | 0.121 | 7.97 |
| 20 | 1.249 | 0.024 | 52.04 | 1.364 | 0.277 | 4.92 |
| 23 | 1.294 | 0.015 | 86.27 | 1.416 | 0.200 | 6.53 |
| 24 | 0.262 | 0.013 | 20.15 | 1.020 | 0.366 | 2.79 |
| 25 | 0.286 | 0.017 | 16.82 | 0.644 | 0.134 | 4.95 |
| 26 | 0.778 | 0.006 | 129.67 | 2.090 | 0.092 | 22.72 |
| 27 | 1.406 | 0.014 | 100.43 | 1.521 | 0.277 | 5.49 |
| 28 | 0.309 | 0.012 | 25.75 | 1.209 | 0.308 | 3.93 |
| 29 | 1.017 | 0.007 | 145.29 | 1.484 | 0.109 | 13.61 |
| 30 | 0.755 | 0.026 | 29.04 | 0.863 | 0.330 | 2.62 |
| 31 | 1.004 | 0.008 | 125.5 | 1.350 | 0.183 | 7.38 |
| 32 | 1.431 | 0.016 | 89.44 | 2.003 | 0.092 | 21.77 |
| 33 | 0.317 | 0.009 | 35.22 | 0.867 | 0.105 | 8.23 |
| 34 | 0.893 | 0.010 | 89.30 | 0.785 | 0.392 | 2.01 |
| 36 | 0.350 | 0.032 | 10.94 | 1.376 | 0.149 | 9.23 |
| 37 | 2.181 | 0.018 | 121.17 | 1.126 | 0.319 | 3.53 |
| 38 | 0.715 | 0.015 | 47.67 | 1.985 | 0.121 | 16.40 |
| 40 | 0.569 | 0.014 | 40.64 | 0.721 | 0.105 | 6.87 |
| 41 | 0.954 | 0.021 | 45.43 | 0.801 | 0.129 | 6.21 |
| 43 | 0.551 | 0.032 | 17.22 | 0.879 | 0.189 | 4.65 |
| 44 | 1.285 | 0.014 | 91.79 | 0.896 | 0.342 | 2.62 |
| 46 | 0.500 | 0.006 | 83.33 | 0.325 | 0.113 | 2.88 |
| 50 | 0.765 | 0.001 | 765 | 0.490 | 0.189 | 2.58 |
| 51 | 0.335 | 0.010 | 33.5 | 0.273 | 0.683 | 0.40 |
| 55 | 0.287 | 0.015 | 19.33 | 0.262 | 0.637 | 0.41 |
| 56 | 0.451 | 0.019 | 23.74 | 0.589 | 0.139 | 4.24 |

| No of control | Activity of MMP-2 | Expression of TIMP-2 | Ratio MMP-2/ TIMP-2 | Activity of MMP-9 | Expression of TIMP-1 | Ratio MMP-9 / TIMP-1 |
|---|---|---|---|---|---|---|
| 3 | No data | 0.008 | No data | No data | 0.134 | No data |
| 4 | 4.515 | 0.008 | 564.4 | 0.654 | 0.080 | 8.18 |
| 9 | 0.869 | 0.011 | 79.0 | 2.018 | 0.342 | 5.90 |
| 10 | 1.069 | 0.009 | 118.8 | 1.103 | 0.095 | 11.60 |
| 12 | 0.185 | 0.009 | 20.6 | 0.543 | 0.082 | 6.62 |
| 14 | 0.171 | 0.102 | 1.7 | 1.732 | 0.287 | 6.03 |
| 21 | 0.031 | 0.036 | 0.9 | 0.890 | 0.171 | 5.20 |
| 22 | 0.194 | 0.033 | 5.9 | 0.787 | 0.500 | 1.57 |
| 35 | 0.217 | 0.017 | 12.8 | 0.376 | 0.451 | 0.83 |
| 39 | 0.223 | 0.016 | 13.9 | 1.258 | 0.189 | 6.66 |
| 42 | 0.639 | 0.015 | 42.6 | 0.787 | 0.660 | 1.19 |
| 45 | 0.240 | 0.006 | 40.0 | 0.183 | 0.268 | 0.68 |
| 47 | 0.709 | 0.005 | 141.8 | 0.589 | 0.109 | 5.40 |
| 48 | 0.554 | 0.015 | 36.9 | 0.698 | 0.225 | 3.10 |
| 49 | 0.447 | 0.019 | 23.5 | 0.375 | 0.210 | 1.79 |
| 52 | 0.788 | 0.007 | 112.6 | 0.236 | 0.177 | 1.33 |
| 53 | 0.745 | 0.002 | 372.5 | 0.355 | 0.203 | 1.75 |
| 54 | 0.153 | 0.028 | 5.5 | 0.269 | 0.574 | 0.47 |
| 57 | 0.558 | 0.008 | 69.8 | 0.408 | 0.785 | 0.52 |
| 58 | 0.137 | 0.009 | 15.2 | 0.517 | 0.354 | 1.46 |
| 59 | 0.107 | 0.011 | 9.7 | 0.459 | 1.072 | 0.43 |
| 60 | 0.130 | 0.010 | 13.0 | 0.997 | 0.732 | 1.36 |

### Example 8: Statistical evaluation

ANOVA (ANalysis Of VAriables), i.e. a test of the statistical significance of the differences among the mean scores of two or more groups on one or more variables, has been used for statistical evaluations. The Tukey HSD procedure was used to conduct *post hoc* pairwise comparisons following mean value ratios from ANOVA, since interclass (between class I, class II, and class III of DRP) differences were found to be statistically significant. The JMP statistical software version 5.1. (SAS institute, Cary, NC, USA) is a suitable software for performing the statistical evaluation.

## Claims

1. A method for evaluation of the risk of a diabetes mellitus (DM) patient to develop diabetic retinopathy (DRP), for early diagnosis of diabetic retinopathy, for determination of the progression risk of an existing diabetic retinopathy, and for determination of the risk to develop secondary complications to DM, which comprises
(a) determining *ex vivo* in a body fluid sample from the patient at least one parameter selected from the group consisting of (i) the expression level of one or more proteins of the group consisting of NF-kappaB, XIAP, TIMP-1, TIMP-2 and Ly-GDI, (ii) the activity of the matrix metalloproteinases MMP-2 and/or MMP-9, and (iii) the ratio MMP-2 activity:TIMP-2 expression level (MMP-2:TIMP-2) and/or MMP-9 activity:TIMP-1 expression level (MMP-9:TIMP-1);
(b) comparing the parameters determined in step (a) with the corresponding mean values of a non-diabetic control group or with the corresponding earlier values of the same patient ("reverence value") and identifying of increased or decreased parameter levels; and
(c) identifying one or more risk factors based on said increased or decreased parameter levels.

2. The method of claim 1, wherein
(i) in step (a) at least three, preferably at least five parameters are determined; and/or
(ii) in step (c) the risk factors are selected from the group consisting of (A) increased Ly-GDI expression, increased MMP-2 activity, increased ratio MMP-2:TIMP-2, increased MMP-9 activity, increased ratio MMP-9:TIMP-1, and (B) decreased TIMP-1 expression, decreased NF-kappaB expression, decreased XIAP expression, and at most 2 times increased, preferably decreased TIMP-2 expression, in comparison to the reference value.

3. The method of claim 1 or 2, wherein a patient showing more than one, preferably at least four, more preferably at least six of the risk factors has an increased risk to develop DRP or that an existing DRP will progress to later stages, especially to later proliferative stages, or that secondary complications will develop.

4. The method of anyone of claims 1 to 3, wherein the patient shows an increased ratio MMP-9:TIMP-1 as one risk factor.

5. The method of anyone of claims 1 to 4, which is for evaluation of the risk of DRP progression in all DRP stages or DRP development and which comprises the determination of one or more, preferably of all, of the parameters of the group consisting of MMP-9 activity, NF-kappaB expression level, TIMP-1 expression level and the calculation of the ratio MMP-9:TIMP-1, and wherein preferably
(i) a ratio MMP-9:TIMP-1 which is at least 1.5 times higher than the reference value;
(ii) a MMP-9 activity level which is at least 1.3 times higher than the reference value; and/or
(iii) a NF-kappaB expression level which is at least 1.5 times lower than the reference value
indicate a risk of DRP progression.

6. The method of claim 5, which additionally comprises the determination of Ly-GDI expression level and/or XIAP expression level, and wherein preferably
(i) a Ly-GDI expression level which is higher than the reference value; and/or
(ii) a XIAP expression level which is at least 1.5 times lower than the reference value
is an additional indicator for the risk of DRP progression, especially for the risk of DRP progression from nonproliferative to proliferative DRP.

7. The method of anyone of claims 1 to 4, which is for evaluation of the risk of DRP progression specifically in late proliferative DRP stages, especially from DRP class II to class III, and which comprises the determination of one or more, preferably of all, of the parameters of the group consisting of MMP-9 and MMP-2 activity, TIMP-1 expression level, TIMP-2 expression level and the calculation of the ratio MMP-2:TIMP-2 and MMP-9:TIMP-1, and wherein preferably
(i) a TIMP-2 level which is equal or less than two times the reference value;
(ii) a TIMP-1 level which is at least 0.5 times lower than the reference value;
(iii) a MMP-2 level which is at least 1.5 times higher than the reference value;
(iv) a ratio MMP-2:TIMP-2 which is at least 1.5 times higher than the reference value; and/or
(v) a ratio MMP-9:TIMP-1 which is at least 2.5 times higher than the reference value
indicate a risk of DRP progression from proliferative DRP to highly proliferative DRP.

8. The method of claim 7, which additionally comprises the determination of NFkappaB expression level, and wherein preferably
(i) a NFkappaB expression level which is at least 3 times lower than the reference value; and/or
(ii) a MMP-9 activity level which is at least 1.5 times higher than the reference value
is an additional indicator for the risk of DRP progression from proliferative DRP to highly proliferative DRP.

9. The method of anyone of claims 1 to 8, wherein
(i) the body fluid is whole blood or a blood component, preferably a component selected from the group consisting of circulating leukocytes, blood plasma and blood serum;
(ii) the determination of expression levels is performed by DNA or RNA detection methods, preferably by real time PCR;
(iii) the determination of MMP activity is performed by means to measure the gelatinase activity of MMP-2 and MMP-9, preferably by zymography or ELISA.

10. A kit for performing the method of any one of claims 1 to 9 comprising reagents and/or devices for
(i) detecting the expression of the genes listed in claim 1, preferably by real-time PCR, especially primer or molecular beacons based PCR, and/or
(ii) determining the activity of MMP-2 and/or MMP-9, preferably by means to measure the gelatinase activity of MMP-2 and MMP-9, by zymography, or by ELISA.


SEQUENCE LISTING

<110>  Universität Bonn

<120>  Method  for early diagnosis of proliferative diabetic retinopathy

<130>  052670ep

<160>  26

<170>  PatentIn version 3.3

<210>  1
<211>  1884
<212>  DNA
<213>  homo sapiens


<220>
<221>  CDS
<222>  (75)..(1202)
<223>  beta actin

<400>  1
ccgcgtccgc cccgcgagca cagagcctcg cctttgccga tccgccgccc gtccacaccc      60

gccgccagct cacc atg gat gat gat atc gcc gcg ctc gtc gtc gac aac      110
                Met Asp Asp Asp Ile Ala Ala Leu Val Val Asp Asn
                1               5                   10

ggc tcc ggc atg tgc aag gcc ggc ttc gcg ggc gac gat gcc ccc cgg      158
Gly Ser Gly Met Cys Lys Ala Gly Phe Ala Gly Asp Asp Ala Pro Arg
        15                  20                  25

gcc gtc ttc ccc tcc atc gtg ggg cgc ccc agg cac cag ggc gtg atg      206
Ala Val Phe Pro Ser Ile Val Gly Arg Pro Arg His Gln Gly Val Met
    30                  35                  40

gtg ggc atg ggt cag aag gat tcc tat gtg ggc gac gag gcc cag agc      254
Val Gly Met Gly Gln Lys Asp Ser Tyr Val Gly Asp Glu Ala Gln Ser
45                  50                  55                  60

aag aga ggc atc ctc acc ctg aag tac ccc atc gag cac ggc atc gtc      302
Lys Arg Gly Ile Leu Thr Leu Lys Tyr Pro Ile Glu His Gly Ile Val
                65                  70                  75

acc aac tgg gac gac atg gag aaa atc tgg cac cac acc ttc tac aat      350
Thr Asn Trp Asp Asp Met Glu Lys Ile Trp His His Thr Phe Tyr Asn
            80                  85                  90

gag ctg cgt gtg gct ccc gag gag cac ccc gtg ctg ctg acc gag gcc      398
Glu Leu Arg Val Ala Pro Glu Glu His Pro Val Leu Leu Thr Glu Ala
        95                  100                 105

ccc ctg aac ccc aag gcc aac cgc gag aag atg acc cag atc atg ttt      446
Pro Leu Asn Pro Lys Ala Asn Arg Glu Lys Met Thr Gln Ile Met Phe
    110                 115                 120

gag acc ttc aac acc cca gcc atg tac gtt gct atc cag gct gtg cta      494
Glu Thr Phe Asn Thr Pro Ala Met Tyr Val Ala Ile Gln Ala Val Leu
125                 130                 135                 140

tcc ctg tac gcc tct ggc cgt acc act ggc atc gtg atg gac tcc ggt      542

Ser Leu Tyr Ala Ser Gly Arg Thr Thr Gly Ile Val Met Asp Ser Gly
                145                 150                 155

gac ggg gtc acc cac act gtg ccc atc tac gag ggg tat gcc ctc ccc      590
Asp Gly Val Thr His Thr Val Pro Ile Tyr Glu Gly Tyr Ala Leu Pro
            160                 165                 170

cat gcc atc ctg cgt ctg gac ctg gct ggc cgg gac ctg act gac tac      638
His Ala Ile Leu Arg Leu Asp Leu Ala Gly Arg Asp Leu Thr Asp Tyr
        175                 180                 185

ctc atg aag atc ctc acc gag cgc ggc tac agc ttc acc acc acg gcc      686
Leu Met Lys Ile Leu Thr Glu Arg Gly Tyr Ser Phe Thr Thr Thr Ala
    190                 195                 200

gag cgg gaa atc gtg cgt gac att aag gag aag ctg tgc tac gtc gcc      734
Glu Arg Glu Ile Val Arg Asp Ile Lys Glu Lys Leu Cys Tyr Val Ala
205                 210                 215                 220

ctg gac ttc gag caa gag atg gcc acg gct gct tcc agc tcc tcc ctg      782
Leu Asp Phe Glu Gln Glu Met Ala Thr Ala Ala Ser Ser Ser Ser Leu
                225                 230                 235

gag aag agc tac gag ctg cct gac ggc cag gtc atc acc att ggc aat      830
Glu Lys Ser Tyr Glu Leu Pro Asp Gly Gln Val Ile Thr Ile Gly Asn
            240                 245                 250

gag cgg ttc cgc tgc cct gag gca ctc ttc cag cct tcc ttc ctg ggc      878
Glu Arg Phe Arg Cys Pro Glu Ala Leu Phe Gln Pro Ser Phe Leu Gly
        255                 260                 265

atg gag tcc tgt ggc atc cac gaa act acc ttc aac tcc atc atg aag      926
Met Glu Ser Cys Gly Ile His Glu Thr Thr Phe Asn Ser Ile Met Lys
    270                 275                 280

tgt gac gtg gac atc cgc aaa gac ctg tac gcc aac aca gtg ctg tct      974
Cys Asp Val Asp Ile Arg Lys Asp Leu Tyr Ala Asn Thr Val Leu Ser
285                 290                 295                 300

ggc ggc acc acc atg tac cct ggc att gcc gac agg atg cag aag gag     1022
Gly Gly Thr Thr Met Tyr Pro Gly Ile Ala Asp Arg Met Gln Lys Glu
                305                 310                 315

atc act gcc ctg gca ccc agc aca atg aag atc aag atc att gct cct     1070
Ile Thr Ala Leu Ala Pro Ser Thr Met Lys Ile Lys Ile Ile Ala Pro
            320                 325                 330

cct gag cgc aag tac tcc gtg tgg atc ggc ggc tcc atc ctg gcc tcg     1118
Pro Glu Arg Lys Tyr Ser Val Trp Ile Gly Gly Ser Ile Leu Ala Ser
        335                 340                 345

ctg tcc acc ttc cag cag atg tgg atc agc aag cag gag tat gac gag     1166
Leu Ser Thr Phe Gln Gln Met Trp Ile Ser Lys Gln Glu Tyr Asp Glu
    350                 355                 360

tcc ggc ccc tcc atc gtc cac cgc aaa tgc ttc tag gcggactatg          1212
Ser Gly Pro Ser Ile Val His Arg Lys Cys Phe
365                 370                 375

acttagttgc gttacaccct ttcttgacaa aacctaactt gcgcagaaaa caagatgaga   1272

ttggcatggc tttatttgtt ttttttgttt tgttttggtt tttttttttt ttttggcttg   1332

actcaggatt taaaaactgg aacggtgaag gtgacagcag tcggttggag cgagcatccc 1392

ccaaagttca caatgtggcc gaggactttg attgcacatt gttgtttttt taatagtcat 1452

tccaaatatg agatgcgttg ttacaggaag tcccttgcca tcctaaaagc caccccactt 1512

ctctctaagg agaatggccc agtcctctcc caagtccaca caggggaggt gatagcattg 1572

ctttcgtgta aattatgtaa tgcaaaattt ttttaatctt cgccttaata cttttttatt 1632

ttgttttatt ttgaatgatg agccttcgtg cccccccttc cccctttttt gtcccccaac 1692

ttgagatgta tgaaggcttt tggtctccct gggagtgggt ggaggcagcc agggcttacc 1752

tgtacactga cttgagacca gttgaataaa agtgcacacc ttaaaaaaaa aaaaaaaaaa 1812

aaaaaaaaaa aaaaaaaaaa aaaaaaaaaa aaaaaaaaaa aaaaaaaaaa aaaaaaaaaa 1872

aaaaaaaaaa aa 1884

<210> 2
<211> 375
<212> PRT
<213> homo sapiens

<400> 2

Met Asp Asp Asp Ile Ala Ala Leu Val Val Asp Asn Gly Ser Gly Met
1               5                   10                  15

Cys Lys Ala Gly Phe Ala Gly Asp Asp Ala Pro Arg Ala Val Phe Pro
            20                  25                  30

Ser Ile Val Gly Arg Pro Arg His Gln Gly Val Met Val Gly Met Gly
        35                  40                  45

Gln Lys Asp Ser Tyr Val Gly Asp Glu Ala Gln Ser Lys Arg Gly Ile
    50                  55                  60

Leu Thr Leu Lys Tyr Pro Ile Glu His Gly Ile Val Thr Asn Trp Asp
65                  70                  75                  80

Asp Met Glu Lys Ile Trp His His Thr Phe Tyr Asn Glu Leu Arg Val
                85                  90                  95

Ala Pro Glu Glu His Pro Val Leu Leu Thr Glu Ala Pro Leu Asn Pro
            100                 105                 110

Lys Ala Asn Arg Glu Lys Met Thr Gln Ile Met Phe Glu Thr Phe Asn
        115                 120                 125

Thr Pro Ala Met Tyr Val Ala Ile Gln Ala Val Leu Ser Leu Tyr Ala
    130                 135                 140

Ser Gly Arg Thr Thr Gly Ile Val Met Asp Ser Gly Asp Gly Val Thr
145                 150                 155                 160

His Thr Val Pro Ile Tyr Glu Gly Tyr Ala Leu Pro His Ala Ile Leu
                165                 170                 175

Arg Leu Asp Leu Ala Gly Arg Asp Leu Thr Asp Tyr Leu Met Lys Ile
            180                 185                 190

Leu Thr Glu Arg Gly Tyr Ser Phe Thr Thr Thr Ala Glu Arg Glu Ile
        195                 200                 205

Val Arg Asp Ile Lys Glu Lys Leu Cys Tyr Val Ala Leu Asp Phe Glu
    210                 215                 220

Gln Glu Met Ala Thr Ala Ala Ser Ser Ser Ser Leu Glu Lys Ser Tyr
225                 230                 235                 240

Glu Leu Pro Asp Gly Gln Val Ile Thr Ile Gly Asn Glu Arg Phe Arg
                245                 250                 255

Cys Pro Glu Ala Leu Phe Gln Pro Ser Phe Leu Gly Met Glu Ser Cys
            260                 265                 270

Gly Ile His Glu Thr Thr Phe Asn Ser Ile Met Lys Cys Asp Val Asp
        275                 280                 285

Ile Arg Lys Asp Leu Tyr Ala Asn Thr Val Leu Ser Gly Gly Thr Thr
    290                 295                 300

Met Tyr Pro Gly Ile Ala Asp Arg Met Gln Lys Glu Ile Thr Ala Leu
305                 310                 315                 320

Ala Pro Ser Thr Met Lys Ile Lys Ile Ile Ala Pro Pro Glu Arg Lys
                325                 330                 335

Tyr Ser Val Trp Ile Gly Gly Ser Ile Leu Ala Ser Leu Ser Thr Phe
            340                 345                 350

Gln Gln Met Trp Ile Ser Lys Gln Glu Tyr Asp Glu Ser Gly Pro Ser
        355                 360                 365

Ile Val His Arg Lys Cys Phe
    370                 375

<210>  3
<211>  2540

<212>  DNA
<213>  homo sapiens


<220>
<221>  CDS
<222>  (34)..(1527)
<223>  XIAP

<400>  3
gaaaaggtgg acaagtccta ttttcaagag aag atg act ttt aac agt ttt gaa      54
gga tct aaa act tgt gta cct gca gac atc aat aag gaa gaa gaa ttt      102
gta gaa gag ttt aat aga tta aaa act ttt gct aat ttt cca agt ggt      150
agt cct gtt tca gca tca aca ctg gca cga gca ggg ttt ctt tat act      198
ggt gaa gga gat acc gtg cgg tgc ttt agt tgt cat gca gct gta gat      246
aga tgg caa tat gga gac tca gca gtt gga aga cac agg aaa gta tcc      294
cca aat tgc aga ttt atc aac ggc ttt tat ctt gaa aat agt gcc acg      342
cag tct aca aat tct ggt atc cag aat ggt cag tac aaa gtt gaa aac      390
tat ctg gga agc aga gat cat ttt gcc tta gac agg cca tct gag aca      438
cat gca gac tat ctt ttg aga act ggg cag gtt gta gat ata tca gac      486
acc ata tac ccg agg aac cct gcc atg tat tgt gaa gaa gct aga tta      534
aag tcc ttt cag aac tgg cca gac tat gct cac cta acc cca aga gag      582
tta gca agt gct gga ctc tac tac aca ggt att ggt gac caa gtg cag      630
tgc ttt tgt tgt ggt gga aaa ctg aaa aat tgg gaa cct tgt gat cgt      678
gcc tgg tca gaa cac agg cga cac ttt cct aat tgc ttc ttt gtt ttg       726
ggc cgg aat ctt aat att cga agt gaa tct gat gct gtg agt tct gat       774
agg aat ttc cca aat tca aca aat ctt cca aga aat cca tcc atg gca       822
gat tat gaa gca cgg atc ttt act ttt ggg aca tgg ata tac tca gtt       870
aac aag gag cag ctt gca aga gct gga ttt tat gct tta ggt gaa ggt       918
gat aaa gta aag tgc ttt cac tgt gga gga ggg cta act gat tgg aag       966
ccc agt gaa gac cct tgg gaa caa cat gct aaa tgg tat cca ggg tgc      1014
aaa tat ctg tta gaa cag aag gga caa gaa tat ata aac aat att cat      1062
tta act cat tca ctt gag gag tgt ctg gta aga act act gag aaa aca      1110
cca tca cta act aga aga att gat gat acc atc ttc caa aat cct atg      1158
gta caa gaa gct ata cga atg ggg ttc agt ttc aag gac att aag aaa      1206
ata atg gag gaa aaa att cag ata tct ggg agc aac tat aaa tca ctt      1254
gag gtt ctg gtt gca gat cta gtg aat gct cag aaa gac agt atg caa      1302
gat gag tca agt cag act tca tta cag aaa gag att agt act gaa gag      1350
cag cta agg cgc ctg caa gag gag aag ctt tgc aaa atc tgt atg gat      1398
aga aat att gct atc gtt ttt gtt cct tgt gga cat cta gtc act tgt     1446
aaa caa tgt gct gaa gca gtt gac aag tgt ccc atg tgc tac aca gtc     1494
att act ttc aag caa aaa att ttt atg tct taa tctaactcta tagtaggcat   1547
gttatgttgt tcttattacc ctgattgaat gtgtgatgtg aactgacttt aagtaatcag   1607
gattgaattc cattagcatt tgctaccaag taggaaaaaa aatgtacatg gcagtgtttt   1667
agttggcaat ataatctttg aatttcttga tttttcaggg tattagctgt attatccatt   1727
ttttttactg ttatttaatt gaaaccatag actaagaata agaagcatca tactataact   1787
gaacacaatg tgtattcata gtatactgat ttaatttcta agtgtaagtg aattaatcat   1847
ctggattttt tattcttttc agataggctt aacaaatgga gctttctgta tataaatgtg   1907
gagattagag ttaatctccc caatcacata atttgttttg tgtgaaaaag gaataaattg   1967
ttccatgctg gtggaaagat agagattgtt tttagaggtt ggttgttgtg ttttaggatt   2027
ctgtccattt tcttgtaaag ggataaacac ggacgtgtgc gaaatatgtt tgtaaagtga   2087
tttgccattg ttgaaagcgt atttaatgat agaatactat cgagccaaca tgtactgaca   2147
tggaaagatg tcagagatat gttaagtgta aaatgcaagt ggcgggacac tatgtatagt   2207
ctgagccaga tcaaagtatg tatgttgtta atatgcatag aacgagagat ttggaaagat   2267
atacaccaaa ctgttaaatg tggtttctct tcggggaggg ggggattggg ggaggggccc   2327
cagaggggtt ttagaggggc cttttcactt tcgacttttt tcattttgtt ctgttcggat   2387
tttttataag tatgtagacc ccgaagggtt ttatgggaac taacatcagt aacctaaccc   2447
ccgtgactat cctgtgctct tcctagggag ctgtgttgtt tcccacccac cacccttccc   2507
tctgaacaaa tgcctgagtg ctggggcact ttg                                2540

<210>  4
<211>  497
<212>  PRT
<213>  homo sapiens

<400>  4

<210>  5
<211>  3001
<212>  DNA
<213>  homo sapiens

<220>

<221>  CDS
<222>  (164)..(2965)
<223>  NF-kappaB

<400>  5
actttcctgc cccttccccg gccaagccca actccggatc tcgctctcca ccggatctca      60

cccgccacac ccggacaggc ggctggagga ggcgggcgtc taaaattctg ggaagcagaa     120

cctggccgga gccactagac agagccgggc ctagcccaga gac atg gag agt tgc       175
                                                Met Glu Ser Cys
                                                1

tac aac cca ggt ctg gat ggt att att gaa tat gat gat ttc aaa ttg       223
Tyr Asn Pro Gly Leu Asp Gly Ile Ile Glu Tyr Asp Asp Phe Lys Leu
5                   10                  15                  20

aac tcc tcc att gtg gaa ccc aag gag cca gcc cca gaa aca gct gat       271
Asn Ser Ser Ile Val Glu Pro Lys Glu Pro Ala Pro Glu Thr Ala Asp
                25                  30                  35

ggc ccc tac ctg gtg atc gtg gaa cag cct aag cag aga ggc ttc cga       319
Gly Pro Tyr Leu Val Ile Val Glu Gln Pro Lys Gln Arg Gly Phe Arg
            40                  45                  50

ttt cga tat ggc tgt gaa ggc ccc tcc cat gga gga ctg ccc ggt gcc       367
Phe Arg Tyr Gly Cys Glu Gly Pro Ser His Gly Gly Leu Pro Gly Ala
        55                  60                  65

tcc agt gag aag ggc cga aag acc tat ccc act gtc aag atc tgt aac       415
Ser Ser Glu Lys Gly Arg Lys Thr Tyr Pro Thr Val Lys Ile Cys Asn
    70                  75                  80

tac gag gga cca gcc aag atc gag gtg gac ctg gta aca cac agt gac       463
Tyr Glu Gly Pro Ala Lys Ile Glu Val Asp Leu Val Thr His Ser Asp
85                  90                  95                  100

cca cct cgt gct cat gcc cac agt ctg gtg ggc aag caa tgc tcg gag       511
Pro Pro Arg Ala His Ala His Ser Leu Val Gly Lys Gln Cys Ser Glu
                105                 110                 115

ctg ggg atc tgc gcc gtt tct gtg ggg ccc aag gac atg act gcc caa       559
Leu Gly Ile Cys Ala Val Ser Val Gly Pro Lys Asp Met Thr Ala Gln
            120                 125                 130

ttt aac aac ctg ggt gtc ctg cat gtg act aag aag aac atg atg ggg       607
Phe Asn Asn Leu Gly Val Leu His Val Thr Lys Lys Asn Met Met Gly
        135                 140                 145

act atg ata caa aaa ctt cag agg cag cgg ctc cgc tct agg ccc cag       655
Thr Met Ile Gln Lys Leu Gln Arg Gln Arg Leu Arg Ser Arg Pro Gln
    150                 155                 160

ggc ctt acg gag gcc gag cag cgg gag ctg gag caa gag gcc aaa gaa       703
Gly Leu Thr Glu Ala Glu Gln Arg Glu Leu Glu Gln Glu Ala Lys Glu
165                 170                 175                 180

ctg aag aag gtg atg gat ctg agt ata gtg cgg ctg cgc ttc tct gcc       751
Leu Lys Lys Val Met Asp Leu Ser Ile Val Arg Leu Arg Phe Ser Ala
                185                 190                 195

ttc ctt aga gcc agt gat ggc tcc ttc tcc ctg ccc ctg aag cca gtc       799
Phe Leu Arg Ala Ser Asp Gly Ser Phe Ser Leu Pro Leu Lys Pro Val

200                 205                 210

acc tcc cag ccc atc cat gat agc aaa tct ccg ggg gca tca aac ctg       847
Thr Ser Gln Pro Ile His Asp Ser Lys Ser Pro Gly Ala Ser Asn Leu
        215                 220                 225

aag att tct cga atg gac aag aca gca ggc tct gtg cgg ggt gga gat       895
Lys Ile Ser Arg Met Asp Lys Thr Ala Gly Ser Val Arg Gly Gly Asp
    230                 235                 240

gaa gtt tat ctg ctt tgt gac aag gtg cag aaa gat gac att gag gtt       943
Glu Val Tyr Leu Leu Cys Asp Lys Val Gln Lys Asp Asp Ile Glu Val
245                 250                 255                 260

cgg ttc tat gag gat gat gag aat gga tgg cag gcc ttt ggg gac ttc       991
Arg Phe Tyr Glu Asp Asp Glu Asn Gly Trp Gln Ala Phe Gly Asp Phe
                265                 270                 275

tct ccc aca gat gtg cat aaa cag tat gcc att gtg ttc cgg aca ccc      1039
Ser Pro Thr Asp Val His Lys Gln Tyr Ala Ile Val Phe Arg Thr Pro
            280                 285                 290

ccc tat cac aag atg aag att gag cgg cct gta aca gtg ttt ctg caa      1087
Pro Tyr His Lys Met Lys Ile Glu Arg Pro Val Thr Val Phe Leu Gln
        295                 300                 305

ctg aaa cgc aag cga gga ggg gac gtg tct gat tcc aaa cag ttc acc      1135
Leu Lys Arg Lys Arg Gly Gly Asp Val Ser Asp Ser Lys Gln Phe Thr
    310                 315                 320

tat tac cct ctg gtg gaa gac aag gaa gag gtg cag cgg aag cgg agg      1183
Tyr Tyr Pro Leu Val Glu Asp Lys Glu Glu Val Gln Arg Lys Arg Arg
325                 330                 335                 340

aag gcc ttg ccc acc ttc tcc cag ccc ttc ggg ggt ggc tcc cac atg      1231
Lys Ala Leu Pro Thr Phe Ser Gln Pro Phe Gly Gly Gly Ser His Met
                345                 350                 355

ggt gga ggc tct ggg ggt gca gcc ggg ggc tac gga gga gct gga gga      1279
Gly Gly Gly Ser Gly Gly Ala Ala Gly Gly Tyr Gly Gly Ala Gly Gly
            360                 365                 370

ggt ggc agc ctc ggt ttc ttc ccc tcc tcc ctg gcc tac agc ccc tac      1327
Gly Gly Ser Leu Gly Phe Phe Pro Ser Ser Leu Ala Tyr Ser Pro Tyr
        375                 380                 385

cag tcc ggc gcg ggc ccc atg cgg tgc tac ccg gga ggc ggg ggc ggg      1375
Gln Ser Gly Ala Gly Pro Met Arg Cys Tyr Pro Gly Gly Gly Gly Gly
    390                 395                 400

gcg cag atg gcc gcc acg gtg ccc agc agg gac tcc ggg gag gaa gcc      1423
Ala Gln Met Ala Ala Thr Val Pro Ser Arg Asp Ser Gly Glu Glu Ala
405                 410                 415                 420

gcg gag ccg agc gcc ccc tcc agg acc ccc cag tgc gag ccg cag gcc      1471
Ala Glu Pro Ser Ala Pro Ser Arg Thr Pro Gln Cys Glu Pro Gln Ala
                425                 430                 435

ccg gag atg ctg cag cga gct cga gag tac aac gcg cgc ctg ttc ggc      1519
Pro Glu Met Leu Gln Arg Ala Arg Glu Tyr Asn Ala Arg Leu Phe Gly
            440                 445                 450

ctg gcg cac gca gcc ccg agc cct act cga cta ctg cgt cac cgc gga      1567

Leu Ala His Ala Ala Pro Ser Pro Thr Arg Leu Leu Arg His Arg Gly
        455                 460                 465

cgc cgc gcg ctg ctg gcg gga cag cgc cac ctg ctg acg gcg cag gac      1615
Arg Arg Ala Leu Leu Ala Gly Gln Arg His Leu Leu Thr Ala Gln Asp
    470                 475                 480

gag aac gga gac aca cca ctg cac cta gcc atc atc cac ggg cag acc      1663
Glu Asn Gly Asp Thr Pro Leu His Leu Ala Ile Ile His Gly Gln Thr
485                 490                 495                 500

agt gtc att gag cag ata gtc tat gtc atc cac cac gcc cag gac ctc      1711
Ser Val Ile Glu Gln Ile Val Tyr Val Ile His His Ala Gln Asp Leu
                505                 510                 515

ggc gtt gtc aac ctc acc aac cac ctg cac cag acg ccc ctg cac ctg      1759
Gly Val Val Asn Leu Thr Asn His Leu His Gln Thr Pro Leu His Leu
            520                 525                 530

gcg gtg atc acg ggg cag acg agt gtg gtg agc ttt ctg ctg cgg gta      1807
Ala Val Ile Thr Gly Gln Thr Ser Val Val Ser Phe Leu Leu Arg Val
        535                 540                 545

ggt gca gac cca gct ctg ctg gat cgg cat gga gac tca gcc atg cat      1855
Gly Ala Asp Pro Ala Leu Leu Asp Arg His Gly Asp Ser Ala Met His
    550                 555                 560

ctg gcg ctg cgg gca ggc gct ggt gct cct gag ctg ctg cgt gca ctg      1903
Leu Ala Leu Arg Ala Gly Ala Gly Ala Pro Glu Leu Leu Arg Ala Leu
565                 570                 575                 580

ctt cag agt gga gct cct gct gtg ccc cag ctg ttg cat atg cct gac      1951
Leu Gln Ser Gly Ala Pro Ala Val Pro Gln Leu Leu His Met Pro Asp
                585                 590                 595

ttt gag gga ctg tat cca gta cac ctg gcg gtc cga gcc cga agc cct      1999
Phe Glu Gly Leu Tyr Pro Val His Leu Ala Val Arg Ala Arg Ser Pro
            600                 605                 610

gag tgc ctg gat ctg ctg gtg gac agt ggg gct gaa gtg gag gcc aca      2047
Glu Cys Leu Asp Leu Leu Val Asp Ser Gly Ala Glu Val Glu Ala Thr
        615                 620                 625

gag cgg cag ggg gga cga aca gcc ttg cat cta gcc aca gag atg gag      2095
Glu Arg Gln Gly Gly Arg Thr Ala Leu His Leu Ala Thr Glu Met Glu
    630                 635                 640

gag ctg ggg ttg gtc acc cat ctg gtc acc aag ctc cgg gcc aac gtg      2143
Glu Leu Gly Leu Val Thr His Leu Val Thr Lys Leu Arg Ala Asn Val
645                 650                 655                 660

aac gct cgc acc ttt gcg gga aac aca ccc ctg cac ctg gca gct gga      2191
Asn Ala Arg Thr Phe Ala Gly Asn Thr Pro Leu His Leu Ala Ala Gly
                665                 670                 675

ctg ggg tac ccg acc ctc acc cgc ctc ctt ctg aag gct ggt gct gac      2239
Leu Gly Tyr Pro Thr Leu Thr Arg Leu Leu Leu Lys Ala Gly Ala Asp
            680                 685                 690

atc cat gct gaa aac gag gag ccc ctg tgc cca ctg cct tca ccc cct      2287
Ile His Ala Glu Asn Glu Glu Pro Leu Cys Pro Leu Pro Ser Pro Pro
        695                 700                 705

acc tct gat agc gac tcg gac tct gaa ggg cct gag aag gac acc cga     2335
Thr Ser Asp Ser Asp Ser Asp Ser Glu Gly Pro Glu Lys Asp Thr Arg
    710                 715                 720

agc agc ttc cgg ggc cac acg cct ctt gac ctc act tgc agc acc ttg     2383
Ser Ser Phe Arg Gly His Thr Pro Leu Asp Leu Thr Cys Ser Thr Leu
725                 730                 735                 740

gtg aag acc ttg ctg cta aat gct gct cag aac acc atg gag cca ccc     2431
Val Lys Thr Leu Leu Leu Asn Ala Ala Gln Asn Thr Met Glu Pro Pro
                745                 750                 755

ctg acc ccg ccc agc cca gca ggg ccg gga ctg tca ctt ggt gat aca     2479
Leu Thr Pro Pro Ser Pro Ala Gly Pro Gly Leu Ser Leu Gly Asp Thr
            760                 765                 770

gct ctg cag aac ctg gag cag ctg cta gac ggg cca gaa gcc cag ggc     2527
Ala Leu Gln Asn Leu Glu Gln Leu Leu Asp Gly Pro Glu Ala Gln Gly
        775                 780                 785

agc tgg gca gag ctg gca gag cgt ctg ggg ctg cgc agc ctg gta gac     2575
Ser Trp Ala Glu Leu Ala Glu Arg Leu Gly Leu Arg Ser Leu Val Asp
    790                 795                 800

acg tac cga cag aca acc tca ccc agt ggc agc ctc ctg cgc agc tac     2623
Thr Tyr Arg Gln Thr Thr Ser Pro Ser Gly Ser Leu Leu Arg Ser Tyr
805                 810                 815                 820

gag ctg gct ggc ggg gac ctg gca ggt cta ctg gag gcc ctg tct gac     2671
Glu Leu Ala Gly Gly Asp Leu Ala Gly Leu Leu Glu Ala Leu Ser Asp
                825                 830                 835

atg ggc cta gag gag gga gtg agg ctg ctg agg ggt cca gaa acc cga     2719
Met Gly Leu Glu Glu Gly Val Arg Leu Leu Arg Gly Pro Glu Thr Arg
            840                 845                 850

gac aag ctg ccc agc aca gag gtg aag gaa gac agt gcg tac ggg agc     2767
Asp Lys Leu Pro Ser Thr Glu Val Lys Glu Asp Ser Ala Tyr Gly Ser
        855                 860                 865

cag tca gtg gag cag gag gca gag aag ctg ggc cca ccc cct gag cca     2815
Gln Ser Val Glu Gln Glu Ala Glu Lys Leu Gly Pro Pro Pro Glu Pro
    870                 875                 880

cca gga ggg ctc tcg cac ggg cac ccc cag cct cag gtg act gac ctg     2863
Pro Gly Gly Leu Ser His Gly His Pro Gln Pro Gln Val Thr Asp Leu
885                 890                 895                 900

ctg cct gcc ccc agc ccc ctt ccc gga ccc cct gta cag cgt ccc cac     2911
Leu Pro Ala Pro Ser Pro Leu Pro Gly Pro Pro Val Gln Arg Pro His
                905                 910                 915

cta ttt caa atc tta ttt aac acc cca cac cca ccc ctc agt tgg gac     2959
Leu Phe Gln Ile Leu Phe Asn Thr Pro His Pro Pro Leu Ser Trp Asp
            920                 925                 930

aaa taa aggattctca tgggaagggg aggaccccga attcct                    3001
Lys

<210>  6
<211>  933

<212>  PRT
<213>  homo sapiens

<400>  6

Met Glu Ser Cys Tyr Asn Pro Gly Leu Asp Gly Ile Ile Glu Tyr Asp
1               5                   10                  15

Asp Phe Lys Leu Asn Ser Ser Ile Val Glu Pro Lys Glu Pro Ala Pro
            20                  25                  30

Glu Thr Ala Asp Gly Pro Tyr Leu Val Ile Val Glu Gln Pro Lys Gln
        35                  40                  45

Arg Gly Phe Arg Phe Arg Tyr Gly Cys Glu Gly Pro Ser His Gly Gly
    50                  55                  60

Leu Pro Gly Ala Ser Ser Glu Lys Gly Arg Lys Thr Tyr Pro Thr Val
65                  70                  75                  80

Lys Ile Cys Asn Tyr Glu Gly Pro Ala Lys Ile Glu Val Asp Leu Val
                85                  90                  95

Thr His Ser Asp Pro Pro Arg Ala His Ala His Ser Leu Val Gly Lys
            100                 105                 110

Gln Cys Ser Glu Leu Gly Ile Cys Ala Val Ser Val Gly Pro Lys Asp
        115                 120                 125

Met Thr Ala Gln Phe Asn Asn Leu Gly Val Leu His Val Thr Lys Lys
    130                 135                 140

Asn Met Met Gly Thr Met Ile Gln Lys Leu Gln Arg Gln Arg Leu Arg
145                 150                 155                 160

Ser Arg Pro Gln Gly Leu Thr Glu Ala Glu Gln Arg Glu Leu Glu Gln
                165                 170                 175

Glu Ala Lys Glu Leu Lys Lys Val Met Asp Leu Ser Ile Val Arg Leu
            180                 185                 190

Arg Phe Ser Ala Phe Leu Arg Ala Ser Asp Gly Ser Phe Ser Leu Pro
        195                 200                 205

Leu Lys Pro Val Thr Ser Gln Pro Ile His Asp Ser Lys Ser Pro Gly
    210                 215                 220

Ala Ser Asn Leu Lys Ile Ser Arg Met Asp Lys Thr Ala Gly Ser Val
225                 230                 235                 240

Arg Gly Gly Asp Glu Val Tyr Leu Leu Cys Asp Lys Val Gln Lys Asp
                245                 250                 255

Asp Ile Glu Val Arg Phe Tyr Glu Asp Asp Glu Asn Gly Trp Gln Ala
            260                 265                 270

Phe Gly Asp Phe Ser Pro Thr Asp Val His Lys Gln Tyr Ala Ile Val
        275                 280                 285

Phe Arg Thr Pro Pro Tyr His Lys Met Lys Ile Glu Arg Pro Val Thr
    290                 295                 300

Val Phe Leu Gln Leu Lys Arg Lys Arg Gly Gly Asp Val Ser Asp Ser
305                 310                 315                 320

Lys Gln Phe Thr Tyr Tyr Pro Leu Val Glu Asp Lys Glu Glu Val Gln
                325                 330                 335

Arg Lys Arg Arg Lys Ala Leu Pro Thr Phe Ser Gln Pro Phe Gly Gly
            340                 345                 350

Gly Ser His Met Gly Gly Gly Ser Gly Gly Ala Ala Gly Gly Tyr Gly
        355                 360                 365

Gly Ala Gly Gly Gly Gly Ser Leu Gly Phe Phe Pro Ser Ser Leu Ala
    370                 375                 380

Tyr Ser Pro Tyr Gln Ser Gly Ala Gly Pro Met Arg Cys Tyr Pro Gly
385                 390                 395                 400

Gly Gly Gly Gly Ala Gln Met Ala Ala Thr Val Pro Ser Arg Asp Ser
                405                 410                 415

Gly Glu Glu Ala Ala Glu Pro Ser Ala Pro Ser Arg Thr Pro Gln Cys
            420                 425                 430

Glu Pro Gln Ala Pro Glu Met Leu Gln Arg Ala Arg Glu Tyr Asn Ala
        435                 440                 445

Arg Leu Phe Gly Leu Ala His Ala Ala Pro Ser Pro Thr Arg Leu Leu
    450                 455                 460

Arg His Arg Gly Arg Arg Ala Leu Leu Ala Gly Gln Arg His Leu Leu
465                 470                 475                 480

Thr Ala Gln Asp Glu Asn Gly Asp Thr Pro Leu His Leu Ala Ile Ile

                485                 490                 495

His Gly Gln Thr Ser Val Ile Glu Gln Ile Val Tyr Val Ile His His
            500                 505                 510

Ala Gln Asp Leu Gly Val Val Asn Leu Thr Asn His Leu His Gln Thr
        515                 520                 525

Pro Leu His Leu Ala Val Ile Thr Gly Gln Thr Ser Val Val Ser Phe
    530                 535                 540

Leu Leu Arg Val Gly Ala Asp Pro Ala Leu Leu Asp Arg His Gly Asp
545                 550                 555                 560

Ser Ala Met His Leu Ala Leu Arg Ala Gly Ala Gly Ala Pro Glu Leu
                565                 570                 575

Leu Arg Ala Leu Leu Gln Ser Gly Ala Pro Ala Val Pro Gln Leu Leu
            580                 585                 590

His Met Pro Asp Phe Glu Gly Leu Tyr Pro Val His Leu Ala Val Arg
        595                 600                 605

Ala Arg Ser Pro Glu Cys Leu Asp Leu Leu Val Asp Ser Gly Ala Glu
    610                 615                 620

Val Glu Ala Thr Glu Arg Gln Gly Gly Arg Thr Ala Leu His Leu Ala
625                 630                 635                 640

Thr Glu Met Glu Glu Leu Gly Leu Val Thr His Leu Val Thr Lys Leu
                645                 650                 655

Arg Ala Asn Val Asn Ala Arg Thr Phe Ala Gly Asn Thr Pro Leu His
            660                 665                 670

Leu Ala Ala Gly Leu Gly Tyr Pro Thr Leu Thr Arg Leu Leu Leu Lys
        675                 680                 685

Ala Gly Ala Asp Ile His Ala Glu Asn Glu Glu Pro Leu Cys Pro Leu
    690                 695                 700

Pro Ser Pro Pro Thr Ser Asp Ser Asp Ser Asp Ser Glu Gly Pro Glu
705                 710                 715                 720

Lys Asp Thr Arg Ser Ser Phe Arg Gly His Thr Pro Leu Asp Leu Thr
                725                 730                 735

Cys Ser Thr Leu Val Lys Thr Leu Leu Leu Asn Ala Ala Gln Asn Thr
            740                 745                 750

Met Glu Pro Pro Leu Thr Pro Pro Ser Pro Ala Gly Pro Gly Leu Ser
        755                 760                 765

Leu Gly Asp Thr Ala Leu Gln Asn Leu Glu Gln Leu Leu Asp Gly Pro
    770                 775                 780

Glu Ala Gln Gly Ser Trp Ala Glu Leu Ala Glu Arg Leu Gly Leu Arg
785                 790                 795                 800

Ser Leu Val Asp Thr Tyr Arg Gln Thr Thr Ser Pro Ser Gly Ser Leu
                805                 810                 815

Leu Arg Ser Tyr Glu Leu Ala Gly Gly Asp Leu Ala Gly Leu Leu Glu
            820                 825                 830

Ala Leu Ser Asp Met Gly Leu Glu Glu Gly Val Arg Leu Leu Arg Gly
        835                 840                 845

Pro Glu Thr Arg Asp Lys Leu Pro Ser Thr Glu Val Lys Glu Asp Ser
    850                 855                 860

Ala Tyr Gly Ser Gln Ser Val Glu Gln Glu Ala Glu Lys Leu Gly Pro
865                 870                 875                 880

Pro Pro Glu Pro Pro Gly Gly Leu Ser His Gly His Pro Gln Pro Gln
                885                 890                 895

Val Thr Asp Leu Leu Pro Ala Pro Ser Pro Leu Pro Gly Pro Pro Val
            900                 905                 910

Gln Arg Pro His Leu Phe Gln Ile Leu Phe Asn Thr Pro His Pro Pro
        915                 920                 925

Leu Ser Trp Asp Lys
    930

<210>  7
<211>  3670
<212>  DNA
<213>  homo sapiens

<220>
<221>  CDS
<222>  (303)..(965)
<223>  TIMP-2

<400> 7
cgcagcaaac acatccgtag aaggcagcgc ggccgccgag aaccgcagcg ccgctcgccc      60

gccgcccccc accccgccgc cccgcccggc gaattgcgcc ccgcgcccct cccctcgcgc     120

ccccgagaca aagaggagag aaagtttgcg cggccgagcg gggcaggtga ggagggtgag     180

ccgcgcggga ggggcccgcc tcggccccgg ctcagccccc gcccgcgccc ccagcccgcc     240

gccgcgagca gcgcccggac cccccagcgg cggcccccgc ccgcccagcc ccccggcccg     300

cc atg ggc gcc gcg gcc cgc acc ctg cgg ctg gcg ctc ggc ctc ctg     347
   Met Gly Ala Ala Ala Arg Thr Leu Arg Leu Ala Leu Gly Leu Leu
   1               5                   10                  15

ctg ctg gcg acg ctg ctt cgc ccg gcc gac gcc tgc agc tgc tcc ccg     395
Leu Leu Ala Thr Leu Leu Arg Pro Ala Asp Ala Cys Ser Cys Ser Pro
                20                  25                  30

gtg cac ccg caa cag gcg ttt tgc aat gca gat gta gtg atc agg gcc     443
Val His Pro Gln Gln Ala Phe Cys Asn Ala Asp Val Val Ile Arg Ala
            35                  40                  45

aaa gcg gtc agt gag aag gaa gtg gac tct gga aac gac att tat ggc     491
Lys Ala Val Ser Glu Lys Glu Val Asp Ser Gly Asn Asp Ile Tyr Gly
        50                  55                  60

aac cct atc aag agg atc cag tat gag atc aag cag ata aag atg ttc     539
Asn Pro Ile Lys Arg Ile Gln Tyr Glu Ile Lys Gln Ile Lys Met Phe
    65                  70                  75

aaa ggg cct gag aag gat ata gag ttt atc tac acg gcc ccc tcc tcg     587
Lys Gly Pro Glu Lys Asp Ile Glu Phe Ile Tyr Thr Ala Pro Ser Ser
80                  85                  90                  95

gca gtg tgt ggg gtc tcg ctg gac gtt gga gga aag aag gaa tat ctc     635
Ala Val Cys Gly Val Ser Leu Asp Val Gly Gly Lys Lys Glu Tyr Leu
                100                 105                 110

att gca gga aag gcc gag ggg gac ggc aag atg cac atc acc ctc tgt     683
Ile Ala Gly Lys Ala Glu Gly Asp Gly Lys Met His Ile Thr Leu Cys
            115                 120                 125

gac ttc atc gtg ccc tgg gac acc ctg agc acc acc cag aag aag agc     731
Asp Phe Ile Val Pro Trp Asp Thr Leu Ser Thr Thr Gln Lys Lys Ser
        130                 135                 140

ctg aac cac agg tac cag atg ggc tgc gag tgc aag atc acg cgc tgc     779
Leu Asn His Arg Tyr Gln Met Gly Cys Glu Cys Lys Ile Thr Arg Cys
    145                 150                 155

ccc atg atc ccg tgc tac atc tcc tcc ccg gac gag tgc ctc tgg atg     827
Pro Met Ile Pro Cys Tyr Ile Ser Ser Pro Asp Glu Cys Leu Trp Met
160                 165                 170                 175

gac tgg gtc aca gag aag aac atc aac ggg cac cag gcc aag ttc ttc     875
Asp Trp Val Thr Glu Lys Asn Ile Asn Gly His Gln Ala Lys Phe Phe
                180                 185                 190

gcc tgc atc aag aga agt gac ggc tcc tgt gcg tgg tac cgc ggc gcg     923
Ala Cys Ile Lys Arg Ser Asp Gly Ser Cys Ala Trp Tyr Arg Gly Ala
            195                 200                 205

gcg ccc ccc aag cag gag ttt ctc gac atc gag gac cca taa          965
Ala Pro Pro Lys Gln Glu Phe Leu Asp Ile Glu Asp Pro
        210                 215                 220

gcaggcctcc aacgcccctg tggccaactg caaaaaaagc ctccaagggt ttcgactggt   1025

ccagctctga catcccttcc tggaaacagc atgaataaaa cactcatccc atgggtccaa   1085

attaatatga ttctgctccc cccttctcct tttagacatg gttgtgggtc tggagggaga   1145

cgtgggtcca aggtcctcat cccatcctcc ctctgccagg cactatgtgt ctggggcttc   1205

gatccttggg tgcaggcagg gctgggacac gcggcttccc tcccagtccc tgccttggca   1265

ccgtcacaga tgccaagcag gcagcactta gggatctccc agctgggtta gggcagggcc   1325

tggaaatgtg cattttgcag aaacttttga gggtcgttgc aagactgtgt agcaggccta   1385

ccaggtccct ttcatcttga gagggacatg gcccttgttt tctgcagctt ccacgcctct   1445

gcactccctg cccctggcaa gtgctcccat cgccccggtg cccaccatga gctcccagca   1505

cctgactccc cccacatcca agggcagcct ggaaccagtg gctagttctt gaaggagccc   1565

catcaatcct attaatcctc agaattccag tgggagcctc cctctgagcc ttgtagaaat   1625

gggagcgaga aaccccagct gagctgcgtt ccagcctcag ctgagtcttt ttggtctgca   1685

cccacccccc cacccccccc cccccgccca catgctcccc agcttgcagg aggaatcggt   1745

gaggtcctgt cctgaggctg ctgtccgggg ccggtggctg ccctcaaggt cccttcccta   1805

gctgctgcgg ttgccattgc ttcttgcctg ttctggcatc aggcacctgg attgagttgc   1865

acagctttgc tttatccggg cttgtgtgca gggcccggct gggctcccca tctgcacatc   1925

ctgaggacag aaaaagctgg gtcttgctgt gccctcccag gcttagtgtt ccctccctca   1985

aagactgaca gccatcgttc tgcacggggc tttctgcatg tgacgccagc taagcatagt   2045

aagaagtcca gcctaggaag ggaaggattt tggaggtagg tggctttggt gacacactca   2105

cttctttctc agcctccagg acactatggc ctgttttaag agacatctta tttttctaaa   2165

ggtgaattct cagatgatag gtgaacctga gttgcagata taccaacttc tgcttgtatt   2225

tcttaaatga caaagattac ctagctaaga aacttcctag ggaactaggg aacctatgtg   2285

ttccctcagt gtggtttcct gaagccagtg atatgggggt taggatagga agaactttct   2345

cggtaatgat aaggagaatc tcttgtttcc tcccacctgt gttgtaaaga taaactgacg   2405

atatacaggc acattatgta aacatacaca cgcaatgaaa ccgaagcttg gcggcctggg   2465

cgtggtcttg caaaatgctt ccaaagccac cttagcctgt tctattcagc ggcaacccca   2525

aagcacctgt taagactcct gacccccaag tggcatgcag cccccatgcc caccgggacc   2585

tggtcagcac agatcttgat gacttccctt tctagggcag actgggaggg tatccaggaa   2645

tcggcccctg ccccacgggc gttttcatgc tgtacagtga cctaaagttg gtaagatgtc   2705

ataatggacc agtccatgtg atttcagtat atacaactcc accagacccc tccaacccat   2765

ataacacccc acccctgttc gcttcctgta tggtgatatc atatgtaaca tttactcctg 2825

tttctgctga ttgttttttt aatgttttgg tttgtttttg acatcagctg taatcattcc 2885

tgtgctgtgt tttttattac ccttggtagg tattagactt gcactttttt aaaaaaaggt 2945

ttctgcatcg tggaagcatt tgacccagag tggaacgcgt ggcctatgca ggtggattcc 3005

ttcaggtctt tcctttggtt ctttgagcat ctttgctttc attcgtctcc cgtctttggt 3065

tctccagttc aaattattgc aaagtaaagg atctttgagt aggttcggtc tgaaaggtgt 3125

ggcctttata tttgatccac acacgttggt cttttaaccg tgctgagcag aaaacaaaac 3185

aggttaagaa gagccgggtg gcagctgaca gaggaagccg ctcaaatacc ttcacaataa 3245

atagtggcaa tatatatata gtttaagaag gctctccatt tggcatcgtt taatttatat 3305

gttatgttct aagcacagct ctcttctcct attttcatcc tgcaagcaac tcaaaatatt 3365

taaaataaag tttacattgt agttattttc aaatctttgc ttgataagta ttaagaaata 3425

ttggacttgc tgccgtaatt taaagctctg ttgattttgt ttccgtttgg atttttgggg 3485

gaggggagca ctgtgtttat gctggaatat gaagtctgag accttccggt gctgggaaca 3545

cacaagagtt gttgaaagtt gacaagcaga ctgcgcatgt ctctgatgct ttgtatcatt 3605

cttgagcaat cgctcggtcc gtggacaata aacagtatta tcaaagagaa aaaaaaaaaa 3665

aaaaa 3670

<210> 8
<211> 220
<212> PRT
<213> homo sapiens

<400> 8

Met Gly Ala Ala Ala Arg Thr Leu Arg Leu Ala Leu Gly Leu Leu Leu
1               5                   10                  15

Leu Ala Thr Leu Leu Arg Pro Ala Asp Ala Cys Ser Cys Ser Pro Val
            20                  25                  30

His Pro Gln Gln Ala Phe Cys Asn Ala Asp Val Val Ile Arg Ala Lys
        35                  40                  45

Ala Val Ser Glu Lys Glu Val Asp Ser Gly Asn Asp Ile Tyr Gly Asn
    50                  55                  60

Pro Ile Lys Arg Ile Gln Tyr Glu Ile Lys Gln Ile Lys Met Phe Lys
65                  70                  75                  80

Gly Pro Glu Lys Asp Ile Glu Phe Ile Tyr Thr Ala Pro Ser Ser Ala
                85                  90                  95

Val Cys Gly Val Ser Leu Asp Val Gly Gly Lys Lys Glu Tyr Leu Ile
            100                 105                 110

Ala Gly Lys Ala Glu Gly Asp Gly Lys Met His Ile Thr Leu Cys Asp
        115                 120                 125

Phe Ile Val Pro Trp Asp Thr Leu Ser Thr Thr Gln Lys Lys Ser Leu
    130                 135                 140

Asn His Arg Tyr Gln Met Gly Cys Glu Cys Lys Ile Thr Arg Cys Pro
145                 150                 155                 160

Met Ile Pro Cys Tyr Ile Ser Ser Pro Asp Glu Cys Leu Trp Met Asp
                165                 170                 175

Trp Val Thr Glu Lys Asn Ile Asn Gly His Gln Ala Lys Phe Phe Ala
            180                 185                 190

Cys Ile Lys Arg Ser Asp Gly Ser Cys Ala Trp Tyr Arg Gly Ala Ala
        195                 200                 205

Pro Pro Lys Gln Glu Phe Leu Asp Ile Glu Asp Pro
    210                 215                 220

<210>  9
<211>  931
<212>  DNA
<213>  homo sapiens

<220>
<221>  CDS
<222>  (193)..(816)
<223>  TIMP-1

<400>  9
tttcgtcggc ccgccccttg gcttctgcac tgatggtggg tggatgagta atgcatccag      60

gaagcctgga ggcctgtggt ttccgcaccc gctgccaccc ccgcccctag cgtggacatt     120

tatcctctag cgctcaggcc ctgccgccat cgccgcagat ccagcgccca gagagacacc     180

agagaaccca cc atg gcc ccc ttt gag ccc ctg gct tct ggc atc ctg ttg     231
              Met Ala Pro Phe Glu Pro Leu Ala Ser Gly Ile Leu Leu
              1               5                   10

ttg ctg tgg ctg ata gcc ccc agc agg gcc tgc acc tgt gtc cca ccc       279
Leu Leu Trp Leu Ile Ala Pro Ser Arg Ala Cys Thr Cys Val Pro Pro
    15                  20                  25

cac cca cag acg gcc ttc tgc aat tcc gac ctc gtc atc agg gcc aag       327
His Pro Gln Thr Ala Phe Cys Asn Ser Asp Leu Val Ile Arg Ala Lys
30                  35                  40                  45

ttc gtg ggg aca cca gaa gtc aac cag acc acc tta tac cag cgt tat 375
Phe Val Gly Thr Pro Glu Val Asn Gln Thr Thr Leu Tyr Gln Arg Tyr
                50                  55                  60

gag atc aag atg acc aag atg tat aaa ggg ttc caa gcc tta ggg gat 423
Glu Ile Lys Met Thr Lys Met Tyr Lys Gly Phe Gln Ala Leu Gly Asp
            65                  70                  75

gcc gct gac atc cgg ttc gtc tac acc ccc gcc atg gag agt gtc tgc 471
Ala Ala Asp Ile Arg Phe Val Tyr Thr Pro Ala Met Glu Ser Val Cys
        80                  85                  90

gga tac ttc cac agg tcc cac aac cgc agc gag gag ttt ctc att gct 519
Gly Tyr Phe His Arg Ser His Asn Arg Ser Glu Glu Phe Leu Ile Ala
    95                  100                 105

gga aaa ctg cag gat gga ctc ttg cac atc act acc tgc agt ttt gtg 567
Gly Lys Leu Gln Asp Gly Leu Leu His Ile Thr Thr Cys Ser Phe Val
110                 115                 120                 125

gct ccc tgg aac agc ctg agc tta gct cag cgc cgg ggc ttc acc aag 615
Ala Pro Trp Asn Ser Leu Ser Leu Ala Gln Arg Arg Gly Phe Thr Lys
                130                 135                 140

acc tac act gtt ggc tgt gag gaa tgc aca gtg ttt ccc tgt tta tcc 663
Thr Tyr Thr Val Gly Cys Glu Glu Cys Thr Val Phe Pro Cys Leu Ser
            145                 150                 155

atc ccc tgc aaa ctg cag agt ggc act cat tgc ttg tgg acg gac cag 711
Ile Pro Cys Lys Leu Gln Ser Gly Thr His Cys Leu Trp Thr Asp Gln
        160                 165                 170

ctc ctc caa ggc tct gaa aag ggc ttc cag tcc cgt cac ctt gcc tgc 759
Leu Leu Gln Gly Ser Glu Lys Gly Phe Gln Ser Arg His Leu Ala Cys
    175                 180                 185

ctg cct cgg gag cca ggg ctg tgc acc tgg cag tcc ctg cgg tcc cag 807
Leu Pro Arg Glu Pro Gly Leu Cys Thr Trp Gln Ser Leu Arg Ser Gln
190                 195                 200                 205

ata gcc tga atcctgcccg gagtggaagc tgaagcctgc acagtgtcca 856
Ile Ala

ccctgttccc actcccatct ttcttccgga caatgaaata aagagttacc acccagcaga 916

aaaaaaaaaa aaaaa 931

<210> 10
<211> 207
<212> PRT
<213> homo sapiens

<400> 10

Met Ala Pro Phe Glu Pro Leu Ala Ser Gly Ile Leu Leu Leu Leu Trp
1               5                   10                  15

Leu Ile Ala Pro Ser Arg Ala Cys Thr Cys Val Pro Pro His Pro Gln
            20                  25                  30

Thr Ala Phe Cys Asn Ser Asp Leu Val Ile Arg Ala Lys Phe Val Gly
        35                  40                  45

Thr Pro Glu Val Asn Gln Thr Thr Leu Tyr Gln Arg Tyr Glu Ile Lys
    50                  55                  60

Met Thr Lys Met Tyr Lys Gly Phe Gln Ala Leu Gly Asp Ala Ala Asp
65                  70                  75                  80

Ile Arg Phe Val Tyr Thr Pro Ala Met Glu Ser Val Cys Gly Tyr Phe
                85                  90                  95

His Arg Ser His Asn Arg Ser Glu Glu Phe Leu Ile Ala Gly Lys Leu
            100                 105                 110

Gln Asp Gly Leu Leu His Ile Thr Thr Cys Ser Phe Val Ala Pro Trp
        115                 120                 125

Asn Ser Leu Ser Leu Ala Gln Arg Arg Gly Phe Thr Lys Thr Tyr Thr
    130                 135                 140

Val Gly Cys Glu Glu Cys Thr Val Phe Pro Cys Leu Ser Ile Pro Cys
145                 150                 155                 160

Lys Leu Gln Ser Gly Thr His Cys Leu Trp Thr Asp Gln Leu Leu Gln
                165                 170                 175

Gly Ser Glu Lys Gly Phe Gln Ser Arg His Leu Ala Cys Leu Pro Arg
            180                 185                 190

Glu Pro Gly Leu Cys Thr Trp Gln Ser Leu Arg Ser Gln Ile Ala
        195                 200                 205

<210>  11
<211>  3065
<212>  DNA
<213>  homo sapiens

<220>
<221>  CDS
<222>  (267)..(2249)
<223>  MMP-2

<400>  11
ctcaggcggt ggctggaggc tgcgcatctg gggctttaaa catacaaagg gattgccagg      60
acctgcggcg gcggcggcgg cggcgggggc tggggcgcgg gggccggacc atgagccgct     120
gagccgggca aaccccaggc caccgagcca gcggaccctc ggagcgcagc cctgcgccgc     180
ggagcaggct ccaaccaggc ggcgacgcgg ccacacgcac cgagccagcg acccccgggc 240
gacgcgcggg gccagggagc gctacg atg gag gcg cta atg gcc cgg ggc gcg 293
ctc acg ggt ccc ctg agg gcg ctc tgt ctc ctg ggc tgc ctg ctg agc 341
cac gcc gcc gcc gcg ccg tcg ccc atc atc aag ttc ccc ggc gat gtc 389
gcc ccc aaa acg gac aaa gag ttg gca gtg caa tac ctg aac acc ttc 437
tat ggc tgc ccc aag gag agc tgc aac ctg ttt gtg ctg aag gac aca 485
cta aag aag atg cag aag ttc ttt gga ctg ccc cag aca ggt gat ctt 533
gac cag aat acc atc gag acc atg cgg aag cca cgc tgc ggc aac cca 581
gat gtg gcc aac tac aac ttc ttc cct cgc aag ccc aag tgg gac aag 629
aac cag atc aca tac agg atc att ggc tac aca cct gat ctg gac cca 677
gag aca gtg gat gat gcc ttt gct cgt gcc ttc caa gtc tgg agc gat 725
gtg acc cca ctg cgg ttt tct cga atc cat gat gga gag gca gac atc 773
atg atc aac ttt ggc cgc tgg gag cat ggc gat gga tac ccc ttt gac 821
ggt aag gac gga ctc ctg gct cat gcc ttc gcc cca ggc act ggt gtt 869
ggg gga gac tcc cat ttt gat gac gat gag cta tgg acc ttg gga gaa 917
ggc caa gtg gtc cgt gtg aag tat ggc aac gcc gat ggg gag tac tgc 965
aag ttc ccc ttc ttg ttc aat ggc aag gag tac aac agc tgc act gat     1013
acc ggc cgc agc gat ggc ttc ctc tgg tgc tcc acc acc tac aac ttt     1061
gag aag gat ggc aag tac ggc ttc tgt ccc cat gaa gcc ctg ttc acc     1109
atg ggc ggc aac gct gaa gga cag ccc tgc aag ttt cca ttc cgc ttc     1157
cag ggc aca tcc tat gac agc tgc acc act gag ggc cgc acg gat ggc     1205
tac cgc tgg tgc ggc acc act gag gac tac gac cgc gac aag aag tat     1253
ggc ttc tgc cct gag acc gcc atg tcc act gtt ggt ggg aac tca gaa     1301
ggt gcc ccc tgt gtc ttc ccc ttc act ttc ctg ggc aac aaa tat gag     1349
agc tgc acc agc gcc ggc cgc agt gac gga aag atg tgg tgt gcg acc     1397
aca gcc aac tac gat gac gac cgc aag tgg ggc ttc tgc cct gac caa     1445
ggg tac agc ctg ttc ctc gtg gca gcc cac gag ttt ggc cac gcc atg     1493
ggg ctg gag cac tcc caa gac cct ggg gcc ctg atg gca ccc att tac     1541
acc tac acc aag aac ttc cgt ctg tcc cag gat gac atc aag ggc att     1589
cag gag ctc tat ggg gcc tct cct gac att gac ctt ggc acc ggc ccc     1637
acc ccc aca ctg ggc cct gtc act cct gag atc tgc aaa cag gac att     1685
gta ttt gat ggc atc gct cag atc cgt ggt gag atc ttc ttc ttc aag     1733
gac cgg ttc att tgg cgg act gtg acg cca cgt gac aag ccc atg ggg     1781
ccc ctg ctg gtg gcc aca ttc tgg cct gag ctc ccg gaa aag att gat     1829
gcg gta tac gag gcc cca cag gag gag aag gct gtg ttc ttt gca ggg     1877
aat gaa tac tgg atc tac tca gcc agc acc ctg gag cga ggg tac ccc     1925
aag cca ctg acc agc ctg gga ctg ccc cct gat gtc cag cga gtg gat     1973
gcc gcc ttt aac tgg agc aaa aac aag aag aca tac atc ttt gct gga     2021
gac aaa ttc tgg aga tac aat gag gtg aag aag aaa atg gat cct ggc     2069
ttt ccc aag ctc atc gca gat gcc tgg aat gcc atc ccc gat aac ctg     2117
gat gcc gtc gtg gac ctg cag ggc ggc ggt cac agc tac ttc ttc aag     2165
ggt gcc tat tac ctg aag ctg gag aac caa agt ctg aag agc gtg aag     2213
ttt gga agc atc aaa tcc gac tgg cta ggc tgc tga gctggccctg          2259
gctcccacag gcccttcctc tccactgcct tcgatacacc gggcctggag aactagagaa   2319
ggacccggag gggcctggca gccgtgcctt cagctctaca gctaatcagc attctcactc   2379
ctacctggta atttaagatt ccagagagtg gctcctcccg gtgcccaaga atagatgctg   2439
actgtactcc tcccaggcgc cccttccccc tccaatccca ccaaccctca gagccacccc   2499
taaagagatc ctttgatatt ttcaacgcag ccctgctttg ggctgccctg gtgctgccac   2559
acttcaggct cttctccttt cacaaccttc tgtggctcac agaacccttg gagccaatgg   2619
agactgtctc aagagggcac tggtggcccg acagcctggc acagggcagt gggacagggc   2679
atggccaggt ggccactcca gacccctggc ttttcactgc tggctgcctt agaacctttc   2739
ttacattagc agtttgcttt gtatgcactt tgtttttttc tttgggtctt gttttttttt   2799
tccacttaga aattgcattt cctgacagaa ggactcaggt tgtctgaagt cactgcacag 2859
tgcatctcag cccacatagt gatggttccc ctgttcactc tacttagcat gtccctaccg 2919
agtctcttct ccactggatg gaggaaaacc aagccgtggc ttcccgctca gccctccctg 2979
cccctccctt caaccattcc ccatgggaaa tgtcaacaag tatgaataaa gacacctact 3039
gagtgaaaaa aaaaaaaaaa aaaaaa 3065

<210> 12
<211> 660
<212> PRT
<213> homo sapiens

<400> 12

<210>  13
<211>  2334
<212>  DNA

<213>  homo sapiens


<220>
<221>  CDS
<222>  (20)..(2143)
<223>  MMP-9

<400>  13
agacacctct gccctcacc atg agc ctc tgg cag ccc ctg gtc ctg gtg ctc      52
                     Met Ser Leu Trp Gln Pro Leu Val Leu Val Leu
                     1               5                   10

ctg gtg ctg ggc tgc tgc ttt gct gcc ccc aga cag cgc cag tcc acc      100
Leu Val Leu Gly Cys Cys Phe Ala Ala Pro Arg Gln Arg Gln Ser Thr
            15                  20                  25

ctt gtg ctc ttc cct gga gac ctg aga acc aat ctc acc gac agg cag      148
Leu Val Leu Phe Pro Gly Asp Leu Arg Thr Asn Leu Thr Asp Arg Gln
        30                  35                  40

ctg gca gag gaa tac ctg tac cgc tat ggt tac act cgg gtg gca gag      196
Leu Ala Glu Glu Tyr Leu Tyr Arg Tyr Gly Tyr Thr Arg Val Ala Glu
    45                  50                  55

atg cgt gga gag tcg aaa tct ctg ggg cct gcg ctg ctg ctt ctc cag      244
Met Arg Gly Glu Ser Lys Ser Leu Gly Pro Ala Leu Leu Leu Leu Gln
60                  65                  70                  75

aag caa ctg tcc ctg ccc gag acc ggt gag ctg gat agc gcc acg ctg      292
Lys Gln Leu Ser Leu Pro Glu Thr Gly Glu Leu Asp Ser Ala Thr Leu
                80                  85                  90

aag gcc atg cga acc cca cgg tgc ggg gtc cca gac ctg ggc aga ttc      340
Lys Ala Met Arg Thr Pro Arg Cys Gly Val Pro Asp Leu Gly Arg Phe
            95                  100                 105

caa acc ttt gag ggc gac ctc aag tgg cac cac cac aac atc acc tat      388
Gln Thr Phe Glu Gly Asp Leu Lys Trp His His His Asn Ile Thr Tyr
        110                 115                 120

tgg atc caa aac tac tcg gaa gac ttg ccg cgg gcg gtg att gac gac      436
Trp Ile Gln Asn Tyr Ser Glu Asp Leu Pro Arg Ala Val Ile Asp Asp
    125                 130                 135

gcc ttt gcc cgc gcc ttc gca ctg tgg agc gcg gtg acg ccg ctc acc      484
Ala Phe Ala Arg Ala Phe Ala Leu Trp Ser Ala Val Thr Pro Leu Thr
140                 145                 150                 155

ttc act cgc gtg tac agc cgg gac gca gac atc gtc atc cag ttt ggt      532
Phe Thr Arg Val Tyr Ser Arg Asp Ala Asp Ile Val Ile Gln Phe Gly
                160                 165                 170

gtc gcg gag cac gga gac ggg tat ccc ttc gac ggg aag gac ggg ctc      580
Val Ala Glu His Gly Asp Gly Tyr Pro Phe Asp Gly Lys Asp Gly Leu
            175                 180                 185

ctg gca cac gcc ttt cct cct ggc ccc ggc att cag gga gac gcc cat      628
Leu Ala His Ala Phe Pro Pro Gly Pro Gly Ile Gln Gly Asp Ala His
        190                 195                 200

ttc gac gat gac gag ttg tgg tcc ctg ggc aag ggc gtc gtg gtt cca      676
Phe Asp Asp Asp Glu Leu Trp Ser Leu Gly Lys Gly Val Val Val Pro

205                 210                 215

act cgg ttt gga aac gca gat ggc gcg gcc tgc cac ttc ccc ttc atc      724
Thr Arg Phe Gly Asn Ala Asp Gly Ala Ala Cys His Phe Pro Phe Ile
220                 225                 230                 235

ttc gag ggc cgc tcc tac tct gcc tgc acc acc gac ggt cgc tcc gac      772
Phe Glu Gly Arg Ser Tyr Ser Ala Cys Thr Thr Asp Gly Arg Ser Asp
                240                 245                 250

ggc ttg ccc tgg tgc agt acc acg gcc aac tac gac acc gac gac cgg      820
Gly Leu Pro Trp Cys Ser Thr Thr Ala Asn Tyr Asp Thr Asp Asp Arg
            255                 260                 265

ttt ggc ttc tgc ccc agc gag aga ctc tac acc cgg gac ggc aat gct      868
Phe Gly Phe Cys Pro Ser Glu Arg Leu Tyr Thr Arg Asp Gly Asn Ala
        270                 275                 280

gat ggg aaa ccc tgc cag ttt cca ttc atc ttc caa ggc caa tcc tac      916
Asp Gly Lys Pro Cys Gln Phe Pro Phe Ile Phe Gln Gly Gln Ser Tyr
    285                 290                 295

tcc gcc tgc acc acg gac ggt cgc tcc gac ggc tac cgc tgg tgc gcc      964
Ser Ala Cys Thr Thr Asp Gly Arg Ser Asp Gly Tyr Arg Trp Cys Ala
300                 305                 310                 315

acc acc gcc aac tac gac cgg gac aag ctc ttc ggc ttc tgc ccg acc     1012
Thr Thr Ala Asn Tyr Asp Arg Asp Lys Leu Phe Gly Phe Cys Pro Thr
                320                 325                 330

cga gct gac tcg acg gtg atg ggg ggc aac tcg gcg ggg gag ctg tgc     1060
Arg Ala Asp Ser Thr Val Met Gly Gly Asn Ser Ala Gly Glu Leu Cys
            335                 340                 345

gtc ttc ccc ttc act ttc ctg ggt aag gag tac tcg acc tgt acc agc     1108
Val Phe Pro Phe Thr Phe Leu Gly Lys Glu Tyr Ser Thr Cys Thr Ser
        350                 355                 360

gag ggc cgc gga gat ggg cgc ctc tgg tgc gct acc acc tcg aac ttt     1156
Glu Gly Arg Gly Asp Gly Arg Leu Trp Cys Ala Thr Thr Ser Asn Phe
    365                 370                 375

gac agc gac aag aag tgg ggc ttc tgc ccg gac caa gga tac agt ttg     1204
Asp Ser Asp Lys Lys Trp Gly Phe Cys Pro Asp Gln Gly Tyr Ser Leu
380                 385                 390                 395

ttc ctc gtg gcg gcg cat gag ttc ggc cac gcg ctg ggc tta gat cat     1252
Phe Leu Val Ala Ala His Glu Phe Gly His Ala Leu Gly Leu Asp His
                400                 405                 410

tcc tca gtg ccg gag gcg ctc atg tac cct atg tac cgc ttc act gag     1300
Ser Ser Val Pro Glu Ala Leu Met Tyr Pro Met Tyr Arg Phe Thr Glu
            415                 420                 425

ggg ccc ccc ttg cat aag gac gac gtg aat ggc atc cgg cac ctc tat     1348
Gly Pro Pro Leu His Lys Asp Asp Val Asn Gly Ile Arg His Leu Tyr
        430                 435                 440

ggt cct cgc cct gaa cct gag cca cgg cct cca acc acc acc aca ccg     1396
Gly Pro Arg Pro Glu Pro Glu Pro Arg Pro Pro Thr Thr Thr Thr Pro
    445                 450                 455

cag ccc acg gct ccc ccg acg gtc tgc ccc acc gga ccc ccc act gtc     1444

Gln Pro Thr Ala Pro Pro Thr Val Cys Pro Thr Gly Pro Pro Thr Val
460                 465                 470                 475

cac ccc tca gag cgc ccc aca gct ggc ccc aca ggt ccc ccc tca gct      1492
His Pro Ser Glu Arg Pro Thr Ala Gly Pro Thr Gly Pro Pro Ser Ala
                480                 485                 490

ggc ccc aca ggt ccc ccc act gct ggc cct tct acg gcc act act gtg      1540
Gly Pro Thr Gly Pro Pro Thr Ala Gly Pro Ser Thr Ala Thr Thr Val
            495                 500                 505

cct ttg agt ccg gtg gac gat gcc tgc aac gtg aac atc ttc gac gcc      1588
Pro Leu Ser Pro Val Asp Asp Ala Cys Asn Val Asn Ile Phe Asp Ala
        510                 515                 520

atc gcg gag att ggg aac cag ctg tat ttg ttc aag gat ggg aag tac      1636
Ile Ala Glu Ile Gly Asn Gln Leu Tyr Leu Phe Lys Asp Gly Lys Tyr
    525                 530                 535

tgg cga ttc tct gag ggc agg ggg agc cgg ccg cag ggc ccc ttc ctt      1684
Trp Arg Phe Ser Glu Gly Arg Gly Ser Arg Pro Gln Gly Pro Phe Leu
540                 545                 550                 555

atc gcc gac aag tgg ccc gcg ctg ccc cgc aag ctg gac tcg gtc ttt      1732
Ile Ala Asp Lys Trp Pro Ala Leu Pro Arg Lys Leu Asp Ser Val Phe
                560                 565                 570

gag gag ccg ctc tcc aag aag ctt ttc ttc ttc tct ggg cgc cag gtg      1780
Glu Glu Pro Leu Ser Lys Lys Leu Phe Phe Phe Ser Gly Arg Gln Val
            575                 580                 585

tgg gtg tac aca ggc gcg tcg gtg ctg ggc ccg agg cgt ctg gac aag      1828
Trp Val Tyr Thr Gly Ala Ser Val Leu Gly Pro Arg Arg Leu Asp Lys
        590                 595                 600

ctg ggc ctg gga gcc gac gtg gcc cag gtg acc ggg gcc ctc cgg agt      1876
Leu Gly Leu Gly Ala Asp Val Ala Gln Val Thr Gly Ala Leu Arg Ser
    605                 610                 615

ggc agg ggg aag atg ctg ctg ttc agc ggg cgg cgc ctc tgg agg ttc      1924
Gly Arg Gly Lys Met Leu Leu Phe Ser Gly Arg Arg Leu Trp Arg Phe
620                 625                 630                 635

gac gtg aag gcg cag atg gtg gat ccc cgg agc gcc agc gag gtg gac      1972
Asp Val Lys Ala Gln Met Val Asp Pro Arg Ser Ala Ser Glu Val Asp
                640                 645                 650

cgg atg ttc ccc ggg gtg cct ttg gac acg cac gac gtc ttc cag tac      2020
Arg Met Phe Pro Gly Val Pro Leu Asp Thr His Asp Val Phe Gln Tyr
            655                 660                 665

cga gag aaa gcc tat ttc tgc cag gac cgc ttc tac tgg cgc gtg agt      2068
Arg Glu Lys Ala Tyr Phe Cys Gln Asp Arg Phe Tyr Trp Arg Val Ser
        670                 675                 680

tcc cgg agt gag ttg aac cag gtg gac caa gtg ggc tac gtg acc tat      2116
Ser Arg Ser Glu Leu Asn Gln Val Asp Gln Val Gly Tyr Val Thr Tyr
    685                 690                 695

gac atc ctg cag tgc cct gag gac tag ggctcccgtc ctgctttgca            2163
Asp Ile Leu Gln Cys Pro Glu Asp
700                 705

gtgccatgta aatccccact gggaccaacc ctggggaagg agccagtttg ccggatacaa    2223

actggtattc tgttctggag gaaagggagg agtggaggtg ggctgggccc tctcttctca    2283

cctttgtttt ttgttggagt gtttctaata aacttggatt ctctaacctt t             2334

<210>  14
<211>  707
<212>  PRT
<213>  homo sapiens

<400>  14

Met Ser Leu Trp Gln Pro Leu Val Leu Val Leu Leu Val Leu Gly Cys
1               5                   10                  15

Cys Phe Ala Ala Pro Arg Gln Arg Gln Ser Thr Leu Val Leu Phe Pro
            20                  25                  30

Gly Asp Leu Arg Thr Asn Leu Thr Asp Arg Gln Leu Ala Glu Glu Tyr
        35                  40                  45

Leu Tyr Arg Tyr Gly Tyr Thr Arg Val Ala Glu Met Arg Gly Glu Ser
    50                  55                  60

Lys Ser Leu Gly Pro Ala Leu Leu Leu Leu Gln Lys Gln Leu Ser Leu
65                  70                  75                  80

Pro Glu Thr Gly Glu Leu Asp Ser Ala Thr Leu Lys Ala Met Arg Thr
                85                  90                  95

Pro Arg Cys Gly Val Pro Asp Leu Gly Arg Phe Gln Thr Phe Glu Gly
            100                 105                 110

Asp Leu Lys Trp His His His Asn Ile Thr Tyr Trp Ile Gln Asn Tyr
        115                 120                 125

Ser Glu Asp Leu Pro Arg Ala Val Ile Asp Asp Ala Phe Ala Arg Ala
    130                 135                 140

Phe Ala Leu Trp Ser Ala Val Thr Pro Leu Thr Phe Thr Arg Val Tyr
145                 150                 155                 160

Ser Arg Asp Ala Asp Ile Val Ile Gln Phe Gly Val Ala Glu His Gly
                165                 170                 175

Asp Gly Tyr Pro Phe Asp Gly Lys Asp Gly Leu Leu Ala His Ala Phe
            180                 185                 190

Pro Pro Gly Pro Gly Ile Gln Gly Asp Ala His Phe Asp Asp Asp Glu

195                 200                 205

Leu Trp Ser Leu Gly Lys Gly Val Val Val Pro Thr Arg Phe Gly Asn
    210                 215                 220

Ala Asp Gly Ala Ala Cys His Phe Pro Phe Ile Phe Glu Gly Arg Ser
225                 230                 235                 240

Tyr Ser Ala Cys Thr Thr Asp Gly Arg Ser Asp Gly Leu Pro Trp Cys
                245                 250                 255

Ser Thr Thr Ala Asn Tyr Asp Thr Asp Asp Arg Phe Gly Phe Cys Pro
            260                 265                 270

Ser Glu Arg Leu Tyr Thr Arg Asp Gly Asn Ala Asp Gly Lys Pro Cys
        275                 280                 285

Gln Phe Pro Phe Ile Phe Gln Gly Gln Ser Tyr Ser Ala Cys Thr Thr
    290                 295                 300

Asp Gly Arg Ser Asp Gly Tyr Arg Trp Cys Ala Thr Thr Ala Asn Tyr
305                 310                 315                 320

Asp Arg Asp Lys Leu Phe Gly Phe Cys Pro Thr Arg Ala Asp Ser Thr
                325                 330                 335

Val Met Gly Gly Asn Ser Ala Gly Glu Leu Cys Val Phe Pro Phe Thr
            340                 345                 350

Phe Leu Gly Lys Glu Tyr Ser Thr Cys Thr Ser Glu Gly Arg Gly Asp
        355                 360                 365

Gly Arg Leu Trp Cys Ala Thr Thr Ser Asn Phe Asp Ser Asp Lys Lys
    370                 375                 380

Trp Gly Phe Cys Pro Asp Gln Gly Tyr Ser Leu Phe Leu Val Ala Ala
385                 390                 395                 400

His Glu Phe Gly His Ala Leu Gly Leu Asp His Ser Ser Val Pro Glu
                405                 410                 415

Ala Leu Met Tyr Pro Met Tyr Arg Phe Thr Glu Gly Pro Pro Leu His
            420                 425                 430

Lys Asp Asp Val Asn Gly Ile Arg His Leu Tyr Gly Pro Arg Pro Glu
        435                 440                 445

Pro Glu Pro Arg Pro Pro Thr Thr Thr Thr Pro Gln Pro Thr Ala Pro
    450                 455                 460

Pro Thr Val Cys Pro Thr Gly Pro Pro Thr Val His Pro Ser Glu Arg
465                 470                 475                 480

Pro Thr Ala Gly Pro Thr Gly Pro Pro Ser Ala Gly Pro Thr Gly Pro
                485                 490                 495

Pro Thr Ala Gly Pro Ser Thr Ala Thr Thr Val Pro Leu Ser Pro Val
            500                 505                 510

Asp Asp Ala Cys Asn Val Asn Ile Phe Asp Ala Ile Ala Glu Ile Gly
        515                 520                 525

Asn Gln Leu Tyr Leu Phe Lys Asp Gly Lys Tyr Trp Arg Phe Ser Glu
    530                 535                 540

Gly Arg Gly Ser Arg Pro Gln Gly Pro Phe Leu Ile Ala Asp Lys Trp
545                 550                 555                 560

Pro Ala Leu Pro Arg Lys Leu Asp Ser Val Phe Glu Glu Pro Leu Ser
                565                 570                 575

Lys Lys Leu Phe Phe Phe Ser Gly Arg Gln Val Trp Val Tyr Thr Gly
            580                 585                 590

Ala Ser Val Leu Gly Pro Arg Arg Leu Asp Lys Leu Gly Leu Gly Ala
        595                 600                 605

Asp Val Ala Gln Val Thr Gly Ala Leu Arg Ser Gly Arg Gly Lys Met
    610                 615                 620

Leu Leu Phe Ser Gly Arg Arg Leu Trp Arg Phe Asp Val Lys Ala Gln
625                 630                 635                 640

Met Val Asp Pro Arg Ser Ala Ser Glu Val Asp Arg Met Phe Pro Gly
                645                 650                 655

Val Pro Leu Asp Thr His Asp Val Phe Gln Tyr Arg Glu Lys Ala Tyr
            660                 665                 670

Phe Cys Gln Asp Arg Phe Tyr Trp Arg Val Ser Ser Arg Ser Glu Leu
        675                 680                 685

Asn Gln Val Asp Gln Val Gly Tyr Val Thr Tyr Asp Ile Leu Gln Cys
    690                 695                 700

Pro Glu Asp
705

<210> 15
<211> 1249
<212> DNA
<213> homo sapiens

<220>
<221> CDS
<222> (153)..(758)
<223> Ly-GDI

<400> 15
ggcacgagcc agggtttcct cttcaagtag gtctaaaaca ttttttttct cattgacttc 60

cttcctgttc taactgccag tactcagaag tcagagttga gagacagagg caccccggac 120

agagacgtga agcactgaat aaatagatca ga atg act gaa aaa gcc cca gag 173
Met Thr Glu Lys Ala Pro Glu
1               5

cca cat gtg gag gag gat gac gat gat gag ctg gac agc aag ctc aat 221
Pro His Val Glu Glu Asp Asp Asp Asp Glu Leu Asp Ser Lys Leu Asn
        10                  15                  20

tat aag cct cca cca cag aag tcc ctg aaa gag ctg cag gaa atg gac 269
Tyr Lys Pro Pro Pro Gln Lys Ser Leu Lys Glu Leu Gln Glu Met Asp
    25                  30                  35

aaa gat gat gag agt cta att aag tac aag aaa acg ctg ctg gga gat 317
Lys Asp Asp Glu Ser Leu Ile Lys Tyr Lys Lys Thr Leu Leu Gly Asp
40                  45                  50                  55

ggt cct gtg gtg aca gat ccg aaa gcc ccc aat gtc gtt gtc acc cgg 365
Gly Pro Val Val Thr Asp Pro Lys Ala Pro Asn Val Val Val Thr Arg
                60                  65                  70

ctc acc ctg gtt tgt gag agt gcc ccg gga cca atc acc atg gac ctt 413
Leu Thr Leu Val Cys Glu Ser Ala Pro Gly Pro Ile Thr Met Asp Leu
            75                  80                  85

act gga gat ctg gaa gcc ctc aaa aag gaa acc att gtg tta aag gaa 461
Thr Gly Asp Leu Glu Ala Leu Lys Lys Glu Thr Ile Val Leu Lys Glu
        90                  95                  100

ggt tct gaa tat aga gtc aaa att cac ttc aaa gtg aac agg gat att 509
Gly Ser Glu Tyr Arg Val Lys Ile His Phe Lys Val Asn Arg Asp Ile
    105                 110                 115

gtg tca ggc ctg aaa tac gtt cag cac acc tac agg act ggg gtg aaa 557
Val Ser Gly Leu Lys Tyr Val Gln His Thr Tyr Arg Thr Gly Val Lys
120                 125                 130                 135

gtg gat aaa gca aca ttt atg gtt ggc agc tat gga cct cgg cct gag 605
Val Asp Lys Ala Thr Phe Met Val Gly Ser Tyr Gly Pro Arg Pro Glu
                140                 145                 150

gag tat gag ttc ctc act cca gtt gag gag gct ccc aag ggc atg ctg 653
Glu Tyr Glu Phe Leu Thr Pro Val Glu Glu Ala Pro Lys Gly Met Leu

155                 160                 165

gcc cga ggc acg tac cac aac aag tcc ttc ttc acc gac gat gac aag      701
Ala Arg Gly Thr Tyr His Asn Lys Ser Phe Phe Thr Asp Asp Asp Lys
        170                 175                 180

caa gac cac ctc agc tgg gag tgg aac ctg tcg att aag aag gag tgg      749
Gln Asp His Leu Ser Trp Glu Trp Asn Leu Ser Ile Lys Lys Glu Trp
    185                 190                 195

aca gaa tga atgcatccac cccgttcccc acccttgcca cctggaagaa              798
Thr Glu
200

ttctctcagg cgtgttcagc accctgtccc tcctccctgt ccacagctgg gtccctcttc    858

aacactgcca catttcctta ttgatcgatc ttttcccacc ctgtcactca acgtggtccc    918

tagaacaaga ggcttaaaac cgggctttca cccaacctgc tccctctgat cctccatcag    978

ggccagatct tccacgtctc catctcagta cacaatcatt taatatttcc ctgtcttacc   1038

cctattcaag caactagagg ccagaaaatg ggcaaattat cactaacagg tctttgactc   1098

aggttccagt agttcattct aatgcctaga ttcttttgtg gttgttgctg gcccaatgag   1158

tccctagtca catcccctgc cagagggagt tcttcttttg tgagagacac tgtaaacgac   1218

acaagagaac aagaataaaa caataactgt g                                  1249

<210>  16
<211>  201
<212>  PRT
<213>  homo sapiens

<400>  16

Met Thr Glu Lys Ala Pro Glu Pro His Val Glu Glu Asp Asp Asp Asp
1               5                   10                  15

Glu Leu Asp Ser Lys Leu Asn Tyr Lys Pro Pro Pro Gln Lys Ser Leu
            20                  25                  30

Lys Glu Leu Gln Glu Met Asp Lys Asp Asp Glu Ser Leu Ile Lys Tyr
        35                  40                  45

Lys Lys Thr Leu Leu Gly Asp Gly Pro Val Val Thr Asp Pro Lys Ala
    50                  55                  60

Pro Asn Val Val Val Thr Arg Leu Thr Leu Val Cys Glu Ser Ala Pro
65                  70                  75                  80

Gly Pro Ile Thr Met Asp Leu Thr Gly Asp Leu Glu Ala Leu Lys Lys
                85                  90                  95

Glu Thr Ile Val Leu Lys Glu Gly Ser Glu Tyr Arg Val Lys Ile His

                100                 105                 110

Phe Lys Val Asn Arg Asp Ile Val Ser Gly Leu Lys Tyr Val Gln His
        115                 120                 125

Thr Tyr Arg Thr Gly Val Lys Val Asp Lys Ala Thr Phe Met Val Gly
    130                 135                 140

Ser Tyr Gly Pro Arg Pro Glu Glu Tyr Glu Phe Leu Thr Pro Val Glu
145                 150                 155                 160

Glu Ala Pro Lys Gly Met Leu Ala Arg Gly Thr Tyr His Asn Lys Ser
                165                 170                 175

Phe Phe Thr Asp Asp Asp Lys Gln Asp His Leu Ser Trp Glu Trp Asn
            180                 185                 190

Leu Ser Ile Lys Lys Glu Trp Thr Glu
        195                 200

<210>  17
<211>  19
<212>  DNA
<213>  artificial sequence

<220>
<223>  forward primer beta actin

<400>  17
gatggtgggc atgggtcag                                                    19

<210>  18
<211>  18
<212>  DNA
<213>  artificial sequence

<220>
<223>  reverse primer beta actin

<400>  18
tggggttcag gggggcct                                                     18

<210>  19
<211>  19
<212>  DNA
<213>  artificial sequence

<220>
<223>  forward primer NF-kappaB

<400>  19
ctacaaccca ggtctggat                                                    19

<210>  20
<211>  21
<212>  DNA
<213>  artificial sequence

<220>
<223>  reverse primer NF-kappaB

<400>  20
gtcactgtgt gtgttaccag g                                                21


<210>  21
<211>  26
<212>  DNA
<213>  artificial sequence

<220>
<223>  forward primer XIAP

<400>  21
gaagatgact tttaacagtt ttgaag                                           26


<210>  22
<211>  22
<212>  DNA
<213>  artificial sequence

<220>
<223>  reverse primer XIAP

<400>  22
aatttgggga tactttcctg tg                                               22


<210>  23
<211>  16
<212>  DNA
<213>  artificial sequence

<220>
<223>  forward primer TIMP-1

<400>  23
   ggcccccttt gagccc                                                       16


<210>  24
<211>  18
<212>  DNA
<213>  artificial sequence

<220>
<223>  reverse primer TIMP-1

<400>  24
ctcaggctgt tccaggga                                                    18


<210>  25
<211>  20
<212>  DNA

<213>  artificial sequence

<220>
<223>  forward primer TIMP-2

<400>  25
tcaccctctg tgacttcatc                                                    20


<210>  26
<211>  19
<212>  DNA
<213>  artificial sequence

<220>
<223>  reverse primer TIMP-2

<400>  26
gtcgagaaac tcctgcttg                                                     19